# FASCICULE DE BREVET EUROPEEN

(11) **EP 3 600 227 B1**
(45) Date de publication et mention de la délivrance du brevet: **12.11.2025**
(21) Numéro de dépôt: 18711344.4
(22) Date de dépôt: 16.03.2018
(51) Int. Cl.: A61K 8/31, A61Q 1/00, A61Q 1/04, A61Q 1/14, A61Q 5/00, A61Q 13/00, A61Q 17/00, A61Q 19/00, A61K 8/81, A61Q 19/08, A61K 8/90, A61K 8/91, A61K 8/04

(54) **COMPOSITION GELIFIEE BIOSOURCÉE**
BIOBASIERTE GELIERTE ZUSAMMENSETZUNG
BIOSOURCED GELLED COMPOSITION

(30) Priorité: 20.03.2017 EP 17305307
(43) Date de publication de la demande: 05.02.2020
(73) Titulaire: TotalEnergies OneTech, 92400 Courbevoie (FR)
(72) Inventeur: SWOBODA, Benjamin, 78630 Orgeval (FR); CONTI, Philippe, 92500 Rueil Malmaison (FR); FRANCIS, Rachida, 60200 Compiègne (FR)
(74) Mandataire: August Debouzy
(86) Numéro de dépôt international: PCT/EP2018/056773
(87) Numéro de publication internationale: WO 2018/172228

(56) Documents cités:
- EP-A1- 1 584 322
- EP-A1- 3 095 838
- WO-A1-00/26285
- WO-A1-02/05760
- WO-A1-2016/030837
- DE-A1- 102011 088 157
- FR-A1- 2 911 497

## Description

### DOMAINE DE L'INVENTION

L'invention concerne une composition gélifiée comprenant au moins une huile hydrocarbonée classée biodégradable, d'origine biologique et majoritairement isoparaffinique.

L'invention concerne également une composition cosmétique, dermatologique ou pharmaceutique comprenant ladite composition gélifiée et son utilisation.

La présente invention concerne aussi l'utilisation de ladite composition gélifiée pour la formulation de produits cosmétiques, de produits dermatologiques ou de produits pharmaceutiques.

### CONTEXTE TECHNIQUE DE L'INVENTION

Les produits cosmétiques existent sous différentes formes. Ils peuvent être soit sous forme d'émulsions (un mélange entre une phase aqueuse et une phase grasse stabilisée par un émulsionnant), soit entièrement aqueux, soit entièrement anhydres (uniquement une phase grasse). Pour texturer ou augmenter la viscosité de la phase grasse, soit dans une émulsion soit dans un produit anhydre, l'homme du métier utilisera un gel de phase grasse qui est un mélange entre une huile apolaire et un gélifiant. Ces gels sont particulièrement utilisés pour la formulation de gloss, d'hydratant pour les lèvres, de crème pour la peau ou de produit solaire. Mais on les retrouve également dans les produits capillaires, les crèmes dépilatoire et les déodorants.

Ces gels peuvent également trouver des applications en pharmacie, en home care et dans l'industrie générale.

Ainsi, un gel apolaire est un mélange d'une huile apolaire et d'un gélifiant. Les huiles apolaires peuvent être des huiles minérales, des huiles végétales, des huiles silicones ou des isoparaffines. Le gélifiant est le plus souvent polymérique tel que le copolymère éthylène/propylène styrène ou le copolymère butylène/éthylène/styrène ou les copolymères polyisobutène, polydécène ou isoprène (ou encore SEBS, SBS, SIS, TPE,...). Ces copolymères sont typiquement vendus par les sociétés Kraton ou Kuraray.

Les gels les plus répandus sont les gels à base d'huile minérale. La compatibilité entre ces huiles et les copolymères est très bonne, en effet la structure apolaire des copolymères est très compatible avec ces huiles. Les inconvénients majeurs de gel à base d'huile minérale sont : l'origine fossile de l'huile et la texture filante du produit.

Le document WO 02/05760 porte sur une composition cosmétique et/ou physiologique comprenant au moins un composé pulvérulent et au moins un matériau pro-adhésif. Le document EP 1 584 322 concerne une composition cosmétique anhydre comprenant un gélifiant polymérique, une huile non volatile et des particules de polyméthacrylate de méthyle. Ces deux documents ne divulguent pas l'huile hydrocarbonée d'origine biologique telle que définie dans la présente invention.

Pour pallier à cet inconvénient (origine fossile ou pétrochimique), des gels à base d'huiles végétales ou d'esters ont été étudiés (par exemple dans la demande US2011/0045983 A1). La compatibilité entre les huiles oxygénées telles que les esters ou les huiles végétales et les copolymères est moins bonne, ce qui peut conduire à des compositions difficiles à formuler. De plus, les esters sont également d'origine chimique. Quant aux huiles végétales (effectivement d'origine biosourcée), elles ont l'inconvénient majeur, en plus de la mauvaise compatibilité avec les copolymères, de rancir, et d'être sensibles à l'oxydation ce qui nécessite généralement l'utilisation d'anti-oxydant. L'instabilité de ces huiles végétales provient notamment de la présence, en quantité substantielle, de molécules comportant des hétéroatomes et/ou des insaturations.

Il subsiste donc le besoin de disposer d'une composition gélifiée (ou gel) comprenant une phase grasse biosourcée présentant une bonne compatibilité entre la phase grasse et le composé gélifiant et qui ne nécessite pas l'ajout d'un antioxydant.

La demanderesse a trouvé de manière surprenante que ce besoin peut être satisfait par une nouvelle composition gélifiée avec une phase grasse d'origine biologique comprenant majoritairement des isoparaffines et permettant de réaliser des compositions stables.

La présente invention a pour objectif de fournir une composition gélifiée isoparaffinique issue de matière première d'origine biologique.

La présente invention a également pour objectif de proposer une composition gélifiée stable aux propriétés adaptées à son utilisation.

### RÉSUMÉ DE L'INVENTION

Ces objectifs sont atteints grâce à une nouvelle composition gélifiée.

L'invention concerne une composition gélifiée comprenant :
- au moins 50% en poids d'au moins une huile hydrocarbonée qui comprend une teneur en poids d'isoparaffines allant de 90 à 100%, une teneur en poids de paraffines normales allant de 0 à 10% et une teneur en carbone d'origine biologique supérieure ou égale à 90 % par rapport au poids total de l'huile hydrocarbonée, et
- au moins 0,5% en poids d'au moins un gélifiant choisi parmi :
   i. les polymères gélifiants choisis parmi les homopolymères ou les copolymères comprenant au moins un monomère choisi parmi l'isoprène, le butadiène, le styrène et les (méth)acrylates,
   ii. les gélifiants non-polymériques,
par rapport au poids total de la composition gélifiée.

Selon un mode de réalisation de l'invention, le gélifiant est un polymère gélifiant choisi parmi les homopolymères ou les copolymères comprenant au moins un monomère choisi parmi l'isoprène, le butadiène, le styrène et les (méth)acrylates.

Selon un mode de réalisation de l'invention, le polymère gélifiant est choisi parmi les homopolymères ou copolymères comprenant au moins un monomère choisi parmi l'isoprène, le butadiène, le styrène.

Selon un mode de réalisation de l'invention, le polymère est un copolymère comprenant au moins un monomère styrène et au moins un monomère choisi parmi l'éthylène, le propylène, le butadiène, l'isoprène.

Selon un mode de réalisation de l'invention, le polymère est choisi parmi les polymères en étoile, les copolymères diblocs, les copolymères triblocs, les copolymères multiblocs, les polymères en peigne, les polymères radiaux, et des combinaisons de ceux-ci.

Selon un mode de réalisation de l'invention, le polymère est choisi parmi les copolymères styrène/butadiène, les copolymères styrène/isoprène, les copolymères styrène/butadiène hydrogénés, les copolymères styrène/isoprène hydrogénés, les polymères croisés polyisoprène hydrogénés, les homopolymères polyisoprène, les homopolymères thermoplastiques de styrène hydrogénés, les caoutchoucs liquides, et leurs mélanges.

Selon un autre mode de réalisation, le gélifiant est un gélifiant non-polymérique choisi parmi les charges minérales, les cires, les sels d'ammonium et les sels métalliques.

Selon un mode de réalisation de l'invention, l'huile hydrocarbonée est choisie parmi les isoparaffines non-cycliques comprenant de 14 à 18 atomes de carbone.

Selon un mode de réalisation de l'invention, l'huile hydrocarbonée comprend :
- une teneur en poids d'isoparaffines allant de 90 à 100%, de préférence de 95 à 100% et préférentiellement de 98% à 100% par rapport au poids total de l'huile hydrocarbonée
- une teneur en carbone d'origine biologique supérieure ou égale à 95%, de préférence supérieure ou égale à 98% et préférentiellement de 100%
- une teneur en poids de paraffines normales inférieure ou égale à 10, de préférence inférieure ou égale à 5% et préférentiellement inférieure ou égale à 2% par rapport au poids total de l'huile hydrocarbonée ; et/ou
- une teneur en poids de composés naphténiques inférieure ou égale à 1%, de préférence inférieure ou égale à 0,5% et préférentiellement inférieure ou égale à 100 ppm par rapport au poids total de l'huile hydrocarbonée ; et/ou
- une teneur en poids de composés aromatiques inférieure ou égale à 500 ppm, de préférence inférieure ou égale à 300 ppm, préférentiellement inférieure ou égale à 100 ppm, plus préférentiellement inférieure ou égale à 50 ppm et avantageusement inférieure ou égale à 20 ppm, par rapport au poids total de l'huile hydrocarbonée.

Selon un mode de réalisation de l'invention, l'huile hydrocarbonée a :
- une température d'ébullition allant de 230 à 340°C, de préférence de 235 à 330°C et plus préférentiellement de 240 à 325°C selon la norme ASTM D86 ; et/ou
- une biodégradabilité à 28 jours d'au moins 60%, de préférence d'au moins 70%, préférentiellement d'au moins 75% et encore plus préférentiellement d'au moins 80% mesurée selon la norme OECD 306 ; et/ou
- un point éclair supérieur ou égal à 110°C selon la norme EN ISO 2719.

Selon un mode de réalisation de l'invention, l'huile hydrocarbonée est obtenue par un procédé d'hydrogénation catalytique à une température de 80 à 180°C et à une pression de 50 à 160 bars d'une charge d'origine biologique désoxygénée et/ou isomérisée.

Selon un mode de réalisation de l'invention, la composition gélifiée comprend, par rapport au poids total de la composition gélifiée, de 50 à 99% en poids, de préférence de 60 à 95% en poids, de préférence encore de 70 à 90% en poids, d'huile hydrocarbonée et de 1 à 50% en poids, de préférence de 5 à 40% en poids, de préférence encore de 10 à 30% en poids, dudit gélifiant, de préférence choisi parmi les polymères gélifiants.

L'invention concerne également une composition cosmétique, dermatologique ou pharmaceutique comprenant au moins une composition gélifiée selon l'invention, de préférence en une quantité allant de 0,5 à 80%, préférentiellement de 1 à 50% et avantageusement de 5 à 30% en poids par rapport au poids total de la composition.

Selon un mode de réalisation de l'invention, la composition cosmétique, dermatologique ou pharmaceutique selon l'invention comprend :
- au moins un corps gras choisi parmi : les huiles végétales, les huiles hydrocarbonées autres que l'huile hydrocarbonée de ladite composition émolliente, les beurres végétaux, les éthers et alcools gras, les esters huileux, les alcanes et les huiles silicones et/ou
- au moins un additif de préférence choisi parmi les émulsionnants.

L'invention a également pour objet l'utilisation de la composition gélifiée selon l'invention, ou de la composition cosmétique, dermatologique ou pharmaceutique selon l'invention pour une application topique, notamment comme produit de soin pour la peau ou des cheveux, comme produit de maquillage, comme produit capillaire, comme produit démaquillant, comme produit parfumé, comme produit solaire, comme produit de soin des lèvres, tel que le gloss ou les sticks hydratants pour les lèvres.

Selon un mode de réalisation de l'invention, la composition gélifiée selon l'invention ou la composition cosmétique, dermatologique ou pharmaceutique est utilisée comme agent anti-âge.

L'invention concerne également un procédé de traitement cosmétique de la peau comprenant au moins une étape d'application, de préférence par étalement, de la composition gélifiée selon l'invention ou de la composition cosmétique, dermatologique ou pharmaceutique selon l'invention.

L'invention a également pour objet une composition gélifiée selon l'invention ou une composition cosmétique, dermatologique ou pharmaceutique selon l'invention, destinée à être utilisée comme médicament.

Selon un mode de réalisation, la composition gélifiée selon l'invention ou la composition cosmétique, dermatologique ou pharmaceutique selon l'invention est destinée à être utilisée en tant qu'antioxydant et/ou agent antiradicalaire et/ou agent anti-inflammatoire et/ou anti-apoptotique et/ou antibactérien et/ou antifongique

### DESCRIPTION DETAILLÉE DE L'INVENTION

L'invention concerne une composition gélifiée comprenant :
- au moins 50% en poids d'au moins une huile hydrocarbonée qui comprend une teneur en poids d'isoparaffines allant de 90 à 100%, une teneur en poids de paraffines normales allant de 0 à 10% et une teneur en carbone d'origine biologique supérieure ou égale à 90 % par rapport au poids total de l'huile hydrocarbonée, et
- au moins 0,5% en poids d'au moins un gélifiant choisi parmi :
   i. les polymères choisis parmi les homopolymères et les copolymères obtenus à partir d'au moins un monomère choisi parmi le styrène, l'isoprène, le butadiène et les (méth)acrylates, et
   ii. les gélifiants non-polymériques choisis de préférence parmi les charges minérales, les cires, les sels d'ammonium et les sels métalliques,
par rapport au poids total de la composition gélifiée.

La composition gélifiée selon l'invention permet de disposer d'une composition classée nonirritante, biodégradable et non-odorante.

La composition gélifiée selon l'invention permet en particulier d'obtenir des compositions cosmétiques, dermatologiques ou pharmaceutiques stables. La stabilité provient notamment du fait que l'huile hydrocarbonée définie dans la présente invention comprend de faibles proportions, voire est substantiellement ou totalement exempte, de composés insaturés et/ou de composés comprenant des hétéroatomes.

A titre préliminaire on notera que, dans la description et les revendications suivantes, l'expression « compris entre » doit s'entendre comme incluant les bornes citées.

### Composition gélifiée :

Selon un mode de réalisation de l'invention, la composition gélifiée selon la présente invention comprend:
- de 50 à 99,5% en poids, de préférence de 60 à 99% en poids, préférentiellement de 70 à 98% en poids, plus préférentiellement de 80 à 95% en poids, encore plus préférentiellement de 85 à 93% en poids, d'huile hydrocarbonée, et
- de 0,5 à 50% en poids, de préférence de 1 à 40% en poids, préférentiellement de 2 à 30% en poids, plus préférentiellement de 5 à 20% en poids, encore plus préférentiellement de 7 à 15% en poids, de gélifiant(s).

Selon un mode de réalisation de l'invention, la composition gélifiée selon la présente invention comprend:
- de 50 à 99,5% en poids, de préférence de 60 à 99% en poids, préférentiellement de 70 à 98% en poids, plus préférentiellement de 80 à 95% en poids, encore plus préférentiellement de 85 à 93% en poids, d'huile hydrocarbonée, et
- de 0,5 à 50% en poids, de préférence de 1 à 40% en poids, préférentiellement de 2 à 30% en poids, plus préférentiellement de 5 à 20% en poids, encore plus préférentiellement de 7 à 15% en poids, de polymère(s) gélifiant(s) choisi(s) parmi les homopolymères et les copolymères comprenant au moins un monomère choisi parmi l'isoprène, le butadiène, le styrène et les (méth)acrylates.

Selon un mode de réalisation de l'invention, la composition gélifiée présente une viscosité mesurée à 23°C (selon une méthode décrite dans la partie expérimentale) allant de 3 à 750000 mPa.s.

Ainsi, la composition gélifiée selon l'invention comprendra de préférence une seule phase grasse.

### Huile hydrocarbonée :

La composition gélifiée selon l'invention comprend de préférence une teneur d'huile hydrocarbonée allant de 50 à 99,5% en poids, préférentiellement de 60 à 99% en poids, plus préférentiellement de 70 à 98% en poids et avantageusement de 80 à 95% en poids par rapport au poids total de la composition.

La présence d'une quantité significative d'huile hydrocarbonée selon l'invention contribue aux qualités cosmétique, pharmaceutique et/ou dermatologique de la composition gélifiée : toucher agréable, soin, brillance et protection de la peau.

L'huile hydrocarbonée de la composition gélifiée selon l'invention comprend de préférence une teneur en poids de composés isoparaffiniques supérieure ou égale à 90%, préférentiellement supérieure ou égale à 95 % et avantageusement supérieure ou égale à 98% par rapport au poids total de l'huile hydrocarbonée.

Selon un mode de réalisation, les composés isoparaffiniques présents dans l'huile hydrocarbonée utilisée selon l'invention comportent de 12 à 30 atomes de carbone, de préférence de 13 à 19 atomes de carbone, de préférence encore de 14 à 18 atomes de carbone.

L'huile hydrocarbonée de la composition gélifiée selon l'invention comprend de préférence une teneur en poids de paraffines normales inférieure ou égale à 10%, préférentiellement inférieure ou égale à 5 % et avantageusement inférieure ou égale à 2%.

L'huile hydrocarbonée de la composition gélifiée selon l'invention comprend avantageusement une majorité d'isoparaffines et une minorité de paraffines normales. Ces isoparaffines sont avantageusement des isoparaffines non-cycliques. De préférence l'huile hydrocarbonée de la composition gélifiée a un ratio massique d'isoparaffines sur paraffines normales d'au moins 12 :1, préférentiellement de 15 :1 et plus préférentiellement de 20 :1. De manière encore plus avantageuse l'huile hydrocarbonée de la composition gélifiée selon l'invention ne contient pas de paraffines normales.

Selon un mode de réalisation, l'huile hydrocarbonée selon l'invention comprend de préférence une teneur en poids d'isoparaffines allant de 90 à 100% et une teneur en paraffines normales allant de 0 à 10%, préférentiellement de 95 à 100% d'isoparaffines et de 0 à 5% de paraffines normales et plus préférentiellement de 98% à 100% d'isoparaffines et de 0 à 2 % de paraffines normales.

Selon un mode de réalisation, l'huile hydrocarbonée de la composition gélifiée selon l'invention comprend de préférence une teneur en poids d'isoparaffines allant de 90 à 100% et une teneur en paraffines normales allant de 0 à 10%, préférentiellement de 95 à 100% d'isoparaffines choisies parmi les alcanes comportant de 12 à 30 atomes de carbone, de préférence de 14 à 18 atomes de carbone, de préférence encore de 14 à 17 atomes de carbone.

Selon un mode de réalisation, l'huile hydrocarbonée mise en œuvre selon l'invention comprend :
- des isoparaffines ayant 15 atomes de carbone et des isoparaffines ayant 16 atomes de carbone en une quantité combinée allant de 80 à 98% en poids, par rapport au poids total de l'huile hydrocarbonée, ou
- des isoparaffines ayant 16 atomes de carbone, des isoparaffines ayant 17 atomes de carbone et des isoparaffines ayant 18 atomes de carbone en une quantité combinée allant de 80 à 98% en poids, par rapport au poids total de l'huile hydrocarbonée, ou
- des isoparaffines ayant 17 atomes de carbone et des isoparaffines ayant 18 atomes de carbone en une quantité combinée allant de 80 à 98% en poids, par rapport au poids total de l'huile hydrocarbonée.

L'huile hydrocarbonée de la composition gélifiée selon l'invention comprend de préférence une teneur en poids de composés naphténiques inférieure ou égale à 1%, préférentiellement inférieure ou égale à 0,5% et plus préférentiellement inférieure ou égale à 100 ppm.

Selon un autre mode de réalisation préféré, l'huile hydrocarbonée de la composition gélifiée selon l'invention comprend une teneur en poids d'isoparaffines allant de 90 à 100%, une teneur en poids de paraffines normales allant de 0 à 10% et une teneur en poids de naphtènes inférieure ou égale à 1%. Préférentiellement l'huile hydrocarbonée comprend une teneur en poids allant de 95 à 100% d'isoparaffines, de 0 à 5% de paraffines normales et une teneur en poids de naphtènes inférieure ou égale à 0,5%. Plus préférentiellement elle comprend une teneur en poids allant de 98% à 100% d'isoparaffines, de 0 à 2 % de paraffines normales et une teneur en poids de naphtènes inférieure ou égale à 100 ppm.

L'huile hydrocarbonée mise en œuvre dans la composition gélifiée selon l'invention est avantageusement exempte de composés aromatiques. Par exempte, on entend une teneur en poids de composés aromatiques inférieure ou égale à 500 ppm, de préférence inférieure ou égale à 300 ppm, préférentiellement inférieure ou égale à 100 ppm, plus préférentiellement inférieure ou égale à 50 ppm et avantageusement inférieure ou égale à 20 ppm mesurée par exemple par spectrométrie UV.

La teneur en poids en isoparaffines, en n-paraffines, en naphtènes et/ou en aromatiques de l'huile hydrocarbonée peut être déterminée selon des méthodes bien connues de l'homme du métier. On peut citer à titre d'exemple non limitatif, une méthode par chromatographie en phase gazeuse.

Selon un autre mode de réalisation préféré, l'huile hydrocarbonée de la composition gélifiée selon l'invention comprend une teneur en poids d'isoparaffines allant de 90 à 100%, une teneur en poids de paraffines normales allant de 0 à 10%, une teneur en poids de naphtènes inférieure ou égale à 1% et une teneur en poids de composés aromatiques inférieure ou égale à 500 ppm. Préférentiellement l'huile hydrocarbonée comprend une teneur en poids allant de 95 à 100% d'isoparaffines, de 0 à 5% de paraffines normales, une teneur en poids de naphtènes inférieure ou égale à 0,5% et une teneur en poids de composés aromatiques inférieure ou égale à 300 ppm, de préférence inférieure à 100 ppm, préférentiellement inférieure à 50 ppm et avantageusement inférieure à 20 ppm. Préférentiellement aussi l'huile hydrocarbonée comprend une teneur en poids allant de 95 à 100% d'isoparaffines, de 0 à 5% de paraffines normales et une teneur en poids de composés aromatiques inférieure ou égale à 100 ppm. Plus préférentiellement elle comprend une teneur en poids allant de 98% à 100% d'isoparaffines, de 0 à 2 % de paraffines normales, une teneur en poids de naphtènes inférieure ou égale à 100 ppm et une teneur en poids de composés aromatiques inférieure ou égale à 100 ppm.

L'huile hydrocarbonée mise en œuvre dans la composition gélifiée selon l'invention a également de préférence une teneur en poids de composés soufrés extrêmement basse, typiquement inférieure ou égale à 5 ppm, préférentiellement inférieure ou égale à 3 ppm et plus préférentiellement inférieure ou égale à 0,5 ppm à un niveau trop bas pour être détectée grâce à des analyseurs de basseteneur en soufre conventionnels.

L'huile hydrocarbonée mise en œuvre dans la composition gélifiée selon l'invention a également de préférence un point éclair supérieur ou égal à 110°C, préférentiellement supérieur ou égal à 120°C et plus préférentiellement supérieur ou égal à 140°C selon la norme EN ISO 2719. Un point éclair élevé, typiquement supérieure à 110°C permettant entre autre de pallier d'une part les problèmes de sécurité lors du stockage et du transport en évitant une inflammabilité trop sensible de l'huile hydrocarbonée.

L'huile hydrocarbonée a aussi de préférence une pression de vapeur à 20°C inférieure ou égale à 0,01kPa.

Selon un mode de réalisation, l'huile hydrocarbonée mise en œuvre dans la composition gélifiée selon l'invention a également de préférence un point éclair supérieur ou égal à 110°C selon la norme EN ISO 2719 et une pression de vapeur à 20°C inférieure ou égale à 0,01kPa. Préférentiellement l'huile hydrocarbonée a un point éclair supérieur ou égal supérieur ou égal à 120°C et une pression de vapeur à 20°C inférieure ou égale à 0,01kPa. Et plus préférentiellement, elle a un point éclair supérieur ou égal à 130°C et une pression de vapeur à 20°C inférieure ou égale à 0,01kPa.

L'huile hydrocarbonée mise en œuvre dans la composition gélifiée selon l'invention présente des températures d'ébullition, un point éclair et une pression de vapeur permettant de pallier les problèmes d'inflammabilité, d'odeur et de volatilité.

L'huile hydrocarbonée de la composition gélifiée selon l'invention a en outre de préférence une viscosité cinématique à 40°C inférieure ou égale à 5 cSt, préférentiellement inférieure ou égale à 4 cSt et plus préférentiellement inférieure ou égale à 3 cSt selon la norme EN ISO 3104.

### Procédé d'obtention de l'huile hydrocarbonée :

De telles compositions d'huiles hydrocarbonées peuvent être obtenues de la façon suivante. L'huile hydrocarbonée selon l'invention est une coupe hydrocarbonée qui est issue de la conversion de la biomasse.

Par issue de la conversion de la biomasse, on entend une coupe hydrocarbonée produite à partir de matières premières d'origine biologique.

De préférence, la coupe hydrocarbonée d'origine biologique est obtenue par un procédé comprenant des étapes d'hydrodésoxygénation (HDO) et d'isomérisation (ISO). L'étape d'hydrodésoxygénation (HDO) conduit à la décomposition des structures des esters biologiques ou des constituants triglycérides, à l'élimination des composés oxygénés, phosphorés et soufrés et à l'hydrogénation des liaisons oléfiniques. Le produit issu de la réaction d'hydrodésoxygénation est ensuite isomérisé. Une étape de fractionnement peut de préférence suivre les étapes d'hydrodésoxygénation et d'isomérisation. De manière avantageuse, les fractions d'intérêt sont ensuite soumises à des étapes d'hydrotraitement puis de distillation afin obtenir les spécifications de l'huile hydrocarbonée souhaitée selon l'invention.

Ce procédé HDO/ISO est mis en œuvre sur une charge biologique brute, encore appelée biomasse ou matière première d'origine biologique, sélectionnée dans le groupe consistant en des huiles végétales, des graisses animales, des huiles de poisson et leur mélange. Les matières premières d'origine biologique appropriées sont par exemple l'huile de colza, l'huile de canola, le talloil, l'huile de tournesol, l'huile de soja, l'huile de chanvre, l'huile d'olive, l'huile de lin, l'huile de moutarde, l'huile de palme, l'huile d'arachide, l'huile de ricin, l'huile de noix de coco, les graisses animales telles que le suif, les graisses alimentaires recyclées, les matières premières issues du génie génétique, et les matières premières biologiques produites à partir de microorganismes tels que les algues et les bactéries. Des produits de condensation, esters ou autres dérivés obtenus à partir de matériaux biologiques bruts peuvent également servir de matières premières.

De préférence, la matière première d'origine biologique est un ester ou un dérivé triglycéride. Ce matériau est soumis tout d'abord à une étape d'hydrodésoxygénation (HDO) pour décomposer la structure des esters ou triglycérides constitutifs et éliminer les composés oxygénés, phosphorés et soufrés de manière concomitante à l'hydrogénation des liaisons oléfiniques. Cette étape d'hydrodésoxygénation (HDO) de la matière première d'origine biologique est suivie par une isomérisation du produit ainsi obtenu conduisant à la ramification de la chaîne hydrocarbonée et à une amélioration des propriétés de la paraffine à basses températures.

Durant l'étape HDO, l'hydrogène et la matière première d'origine biologique sont passés sur un lit catalytique d'hydrodésoxygénation de manière simultanée ou à contre-courant. Durant l'étape HDO, la pression et la température sont comprises entre 20 et 150 bars et entre 200 et 500°C respectivement. Des catalyseurs classiques et connus d'hydrodésoxygénation sont utilisés durant cette étape. Éventuellement, la matière première d'origine biologique peut être soumise à une pré-hydrogénation sous conditions douces pour éviter les réactions secondaires des doubles liaisons avant l'étape HDO. Après l'étape d'hydrodésoxygénation, le produit issu de la réaction est soumis à une étape d'isomérisation (ISO) où l'hydrogène et le produit, et éventuellement un mélange de n-paraffines, sont passés sur des lits catalytiques d'isomérisation de manière simultanée ou à contre-courant. Lors de l'étape ISO, la pression et la température sont comprises entre 20 et 150 bars et entre 200 et 500°C respectivement. Des catalyseurs classiques et connus d'isomérisation sont utilisés durant cette étape.

Des procédés secondaires additionnels peuvent également être mise en œuvre (comme des mélanges intermédiaires, des piégeages ou autres procédés de la sorte).

Le produit issu des étapes HDO/ISO peut éventuellement être fractionné afin d'obtenir les coupes d'intérêt.

Divers procédés HDO/ISO sont décrits dans la littérature. La demande WO2014/033762 décrit un procédé comprenant une étape de pré-hydrogénation, une étape d'hydrodésoxygénation (HDO) et une étape d'isomérisation opérées à contre-courant. La demande de brevet EP1728844 décrit un procédé de production de composés hydrocarbonés à partir d'un mélange de composés d'origine végétale et animale. Ce procédé comprend une étape de prétraitement du mélange permettant d'enlever les contaminants, comme par exemple les sels de métaux alcalins, suivie d'une étape d'hydrodésoxygénation (HDO) et d'une étape d'isomérisation. La demande de brevet EP2084245 décrit un procédé de production d'un mélange hydrocarboné qui peut être utilisé comme gazole ou dans une composition de gazole par hydrodésoxygénation d'un mélange d'origine biologique contenant des esters d'acides gras éventuellement en mélange avec des acides gras libres, par exemple des huiles végétales comme l'huile de tournesol, l'huile de colza, l'huile de canola, l'huile de palme ou l'huile de pin, suivi d'une hydroisomérisation sur des catalyseurs spécifiques. La demande de brevet EP2368967 décrit un tel procédé et le produit obtenu par ce procédé. La demande WO2016/185046 décrit un procédé d'obtention d'une huile hydrocarbonée utilisée selon l'invention, dans lequel l'huile hydrocarbonée est obtenue par un procédé d'hydrogénation catalytique à une température de 80 à 180°C et à une pression de 50 à 160 bars d'une charge d'origine biologique désoxygénée et isomérisée.

Avantageusement, la matière première d'origine biologique contient moins de 15 ppm de soufre, de préférence moins de 8 ppm, préférentiellement moins de 5 ppm et plus préférentiellement moins de 1 ppm selon la norme EN ISO 20846. Idéalement la charge ne comprend pas de soufre en tant que matière première d'origine biosourcée.

Avant l'étape d'hydrotraitement, une étape de préfractionnement peut avoir lieu. Une coupe plus étroite en entrée d'unité d'hydrogénation permet d'obtenir une coupe étroite en sortie d'unité. En effet, les points d'ébullition de coupes préfractionnées sont compris entre 220 et 330 °C tandis que les coupes qui n'ont pas été préfractionnées ont typiquement des points d'ébullition comprise entre 150 et 360°C.

La charge désoxygénée et isomérisée issue du procédé HDO/ISO est ensuite hydrogénée.

L'hydrogène utilisé dans l'unité d'hydrogénation est typiquement de l'hydrogène hautement purifié. On entend par hautement purifié, de l'hydrogène d'une pureté par exemple supérieure à 99%, même si d'autres grades peuvent également être utilisés.

L'étape d'hydrogénation est effectuée grâce à des catalyseurs. Les catalyseurs d'hydrogénation types peuvent être soit massiques soit supportés et peuvent comprendre les métaux suivants : nickel, platine, palladium, rhénium, rhodium, tungstate de nickel, nickel-molybdène, molybdène, cobalt-molybdène. Les supports peuvent être de la silice, de l'alumine, de la silicealumine ou des zéolithes.

Un catalyseur préféré est un catalyseur à base de nickel sur support d'alumine dont l'aire de surface spécifique varie entre 100 et 200 m²/g de catalyseur ou un catalyseur massique à base de nickel. Les conditions d'hydrogénation sont typiquement les suivantes :
- Pression : 50 à 160 bars, de préférence 80 à 150 bars et plus préférentiellement 90 à 120 bars ;
- Température : 80 à 180 °C, de préférence 120 à 160 °C et plus préférentiellement 150 à 160 °C ;
- Vitesse volumique horaire (VVH): 0,2 à 5 hr⁻¹, de préférence 0,4 à 3 hr⁻¹ et plus préférentiellement 0,5 à 0,8 hr⁻¹;
- Taux de traitement par l'hydrogène : adapté aux conditions mentionnées ci-dessus et pouvant aller jusqu'à 200 Nm³/tonnes de charge à traiter.

La température dans les réacteurs est typiquement comprise entre 150 et 160°C avec une pression d'environ 100 bars tandis que la vitesse volumique horaire est d'environ 0,6 hr⁻¹ avec un taux de traitement adapté en fonction de la qualité de la charge à traiter et des paramètres du premier réacteur d'hydrogénation.

L'hydrogénation peut avoir lieu dans un ou plusieurs réacteurs en série. Les réacteurs peuvent comprendre un ou plusieurs lits catalytiques. Les lits catalytiques sont généralement des lits catalytiques fixes.

Le procédé d'hydrogénation comprend de préférence deux ou trois réacteurs, de préférence trois réacteurs et est plus préférentiellement réalisé dans trois réacteurs en série.

Le premier réacteur permet le piégeage des composés soufrés et l'hydrogénation d'essentiellement tous les composés insaturés et jusqu'à environ 90 % des composés aromatiques. Le produit issu du premier réacteur ne contient substantiellement aucun composé soufré. Au second stade c'est-à-dire dans le second réacteur, l'hydrogénation des aromatiques se poursuit et jusqu'à 99 % des aromatiques sont de ce fait hydrogénés.

Le troisième stade dans le troisième réacteur est un stade de finition permettant d'obtenir des teneurs en aromatiques inférieures ou égales à 500 ppm, de préférence inférieures ou égales à 300 ppm, préférentiellement inférieures ou égales à 100 ppm, plus préférentiellement inférieures ou égales à 50 ppm, et idéalement inférieures ou égales à 20 ppm même dans le cas de produits à haut point d'ébullition par exemple supérieur à 300°C.

Il est possible d'utiliser un réacteur qui comporte deux ou trois lits catalytiques ou plus. Les catalyseurs peuvent être présents à des quantités variables ou essentiellement égales dans chaque réacteur ; pour trois réacteurs, les quantités en fonction du poids peuvent par exemple être de 0,05-0,5/0,10-0,70/0,25-0,85, de préférence 0,07-0,25/0,15-0,35/0,4-0,78 et plus préférentiellement de 0,10-0,20/0,20-0,32/0,48-0,70.

Il est également possible d'utiliser un ou deux réacteurs d'hydrogénation au lieu de trois.

Il est également possible que le premier réacteur soit composé de réacteurs jumeaux mis en œuvre de manière alternative. Ce mode d'opérabilité permet notamment un chargement et un déchargement facilité des catalyseurs : lorsque le premier réacteur comprend le catalyseur saturé en premier (substantiellement tout le soufre est piégé sur et/ou dans le catalyseur) il doit être changé souvent.

Un seul réacteur peut également être utilisé dans lequel deux, trois lits catalytiques ou plus sont installés.

Il peut être nécessaire d'insérer des boîtes de quench (au sens anglais « d'étouffement de la réaction ») dans le système de recycle ou entre les réacteurs pour refroidir les effluents d'un réacteur à un autre ou d'un lit catalytique à un autre afin de contrôler les températures et l'équilibre hydrothermique de chaque réaction. Selon un mode de réalisation préféré, il n'y a pas d'intermédiaires de refroidissement ou d'étouffement.

Selon un mode de réalisation, le produit issu du procédé et/ou les gaz séparés sont au moins en partie recyclé(s) dans le système d'alimentation des réacteurs d'hydrogénation. Cette dilution contribue à maintenir l'exothermicité de la réaction dans des limites contrôlées, en particulier au premier stade. Le recyclage permet en outre un échange de chaleur avant la réaction et aussi un meilleur contrôle de la température.

L'effluent de l'unité d'hydrogénation contient principalement le produit hydrogéné et de l'hydrogène. Des séparateurs flash sont utilisés pour séparer les effluents en phase gazeuse, principalement l'hydrogène résiduel, et en phase liquide, principalement les coupes hydrocarbonées hydrogénées. Le procédé peut être effectué en utilisant trois séparateurs flash, un à pression élevée, un à pression intermédiaire et un à basse pression très proche de la pression atmosphérique.

L'hydrogène gazeux qui est recueilli en haut des séparateurs flash peut être recyclé dans le système d'alimentation de l'unité d'hydrogénation ou à différents niveaux dans les unités d'hydrogénation entre les réacteurs.

Selon un mode de réalisation, le produit final est séparé à pression atmosphérique. Il alimente ensuite directement une unité de fractionnement sous vide. De préférence, le fractionnement se fera à une pression comprise entre 10 et 50 mbars et plus préférentiellement à environ 30 mbars.

Le fractionnement peut être effectué de façon à ce qu'il soit possible de retirer simultanément divers fluides hydrocarbonés de la colonne de fractionnement et à ce que leur température d'ébullition puisse être prédéterminée.

En adaptant la charge au travers de ses points d'ébullition initiaux et finaux, les réacteurs d'hydrogénation, les séparateurs et l'unité de fractionnement peuvent donc être directement connectés sans qu'il soit nécessaire d'utiliser des cuves intermédiaires. Cette intégration de l'hydrogénation et du fractionnement permet une intégration thermique optimisée associée à une réduction du nombre d'appareils et à une économie d'énergie.

L'huile hydrocarbonée mise en œuvre dans la composition gélifiée de l'invention est avantageusement une coupe hydrocarbonée ayant un intervalle de distillation ID (en °C) allant de 230°C à 340°C, de préférence de 235°C à 330°C et plus préférentiellement de 240°C à 325°C mesuré selon la norme ASTM D86. De préférence, la différence entre le point d'ébullition initial et le point d'ébullition final est inférieure ou égale à 80°C, préférentiellement inférieure ou égale à 70°C, plus préférentiellement inférieure ou égale à 60°C et avantageusement comprise entre 40 et 50°C. L'huile hydrocarbonée peut comprendre une ou plusieurs fractions d'intervalles de distillation compris dans les intervalles décrits ci-dessus.

Avantageusement, l'huile hydrocarbonée mise en œuvre dans la composition gélifiée de l'invention est totalement saturée. De préférence, les composants de l'huile hydrocarbonée sont choisis parmi les isoparaffines comprenant 12 à 30 atomes de carbone, préférentiellement 13 à 19 atomes de carbone et plus préférentiellement 14 à 18 atomes de carbones.

La composition gélifiée selon l'invention comprend avantageusement une teneur en poids en isohexadécane inférieure ou égale à 50%.

L'huile hydrocarbonée de la composition gélifiée selon l'invention est idéalement issue du traitement de matières premières d'origine biologique. Le terme de « bio-carbone » indique que le carbone est d'origine naturelle et vient d'un biomatériau, comme indiqué ci-après. La teneur en bio-carbone et la teneur en biomatériau sont des expressions indiquant la même valeur. Un matériau d'origine renouvelable ou biomatériau est un matériau organique dans lequel le carbone est issu du CO₂ fixé récemment (sur une échelle humaine) par photosynthèse à partir de l'atmosphère. Un biomatériau (Carbone 100% d'origine naturelle) présente un rapport isotopique ¹⁴C /¹²C supérieur à 10⁻¹², typiquement environ 1,2 x 10⁻¹², tandis qu'un matériau fossile a un rapport nul. En effet, le ¹⁴C isotopique est formé dans l'atmosphère et est alors intégré par photosynthèse, selon une échelle de temps de quelques dizaines d'années au maximum. La demi-vie du ¹⁴C est 5730 années. Ainsi, les matériaux issus de la photosynthèse, à savoir les plantes d'une manière générale, ont nécessairement un contenu maximum en isotope ¹⁴C.

La détermination de la teneur en biomatériau ou en bio-carbone est donnée conformément aux normes ASTM D 6866-12, la méthode B (ASTM D 6866-06) et ASTM D 7026 (ASTM D 7026-04). L'huile hydrocarbonée de la composition gélifiée selon l'invention présente une teneur en biomatériau d'au moins 90%. Cette teneur est avantageusement plus élevée, en particulier supérieure ou égale à 95%, préférablement supérieure ou égale à 98 %et avantageusement égale à 100%.

En plus d'une teneur particulièrement élevée en biomatériau, l'huile hydrocarbonée de la composition gélifiée selon l'invention possède une biodégradabilité particulièrement bonne. La biodégradation d'un produit chimique organique se réfère à la réduction de la complexité des composés chimiques grâce à l'activité métabolique de micro-organismes. Dans des conditions aérobies, les micro-organismes transforment les substances organiques en dioxyde de carbone, eau et biomasse. La méthode OCDE 306, est utilisée pour l'évaluation de la biodégradabilité des substances individuelles dans l'eau de mer. Selon cette méthode, l'huile hydrocarbonée a une biodégradabilité à 28 jours d'au moins 60%, de préférence d'au moins 70%, plus préférablement d'au moins 75% et avantageusement d'au moins 80%.

### Gélifiant(s) de type polymère(s) et non-polymère(s) :

La composition gélifiée selon l'invention peut comprendre un ou plusieurs gélifiants, de type polymères gélifiants ou gélifiants non-polymériques, permettant ainsi de gélifier l'huile hydrocarbonée.

Par « polymère gélifiant », au sens de la présente invention, il faut comprendre un polymère capable de former une composition gélifiée ou gel à partir d'une huile apolaire incluant les huiles hydrocarbonées définies dans l'invention.

Par « gélifiant non-polymérique », au sens de la présente invention, il faut comprendre un composé différent d'un polymère et qui est capable de former une composition gélifiée ou gel à partir d'une huile apolaire incluant les huiles hydrocarbonées définies dans l'invention.

Le ou les gélifiants peuvent représenter de 0,5 à 50% en poids, de préférence de 1 à 40% en poids, de préférence encore de 5 à 30% en poids, du poids total de la composition gélifiée.

Le polymère gélifiant mis en œuvre dans la composition gélifiée selon un mode de réalisation de l'invention est choisi parmi les homopolymères et les copolymères comprenant au moins un monomère choisi parmi l'isoprène, le butadiène, le styrène et les (méth)acrylates, et leurs mélanges.

Par « homopolymère », au sens de la présente invention, il faut comprendre un polymère obtenu à partir d'un seul et même monomère.

Par « copolymère », au sens de la présente invention, il faut comprendre un polymère obtenu à partir d'au moins deux monomères différents.

Par « homopolymères comprenant au moins un monomère choisi parmi l'isoprène, le butadiène, le styrène et les (méth)acrylates », au sens de la présente invention, il faut comprendre les polyisoprènes, les polybutadiènes, les polystyrènes et les homopolymères de (méth)acrylates.

Par « les copolymères comprenant au moins un monomère choisi parmi l'isoprène, le butadiène, le styrène et les (méth)acrylates », au sens de la présente invention, il faut comprendre les copolymères obtenus à partir d'au moins un monomère choisi parmi l'isoprène, le butadiène, le styrène et les (méth)acrylates. Les copolymères mis en œuvre dans la composition gélifiée selon l'invention peuvent comprendre d'autres types de monomères.

Le polymère mis en œuvre dans la composition gélifiée selon un mode de réalisation de l'invention peut se présenter par exemple sous la forme d'une solution, d'une suspension aqueuse ou d'une dispersion.

Selon un mode de réalisation, le polymère est choisi parmi les polymères en étoile, les copolymères diblocs, les copolymères triblocs, les copolymères multiblocs, les polymères en peigne, les polymères radiaux, et des combinaisons de ceux-ci.

Selon un mode de réalisation de l'invention, le polymère gélifiant est choisi parmi les homopolymères ou copolymères comprenant au moins un monomère choisi parmi l'isoprène, le butadiène, le styrène.

Selon un mode de réalisation, le polymère gélifiant comprend au moins un monomère styrène. Selon un mode de réalisation, la teneur en poids en motif styrénique du polymère mis en œuvre selon l'invention va de 5 à 50%, de préférence de 10 à 40%, par rapport au poids total du polymère.

Selon un mode de réalisation, le polymère gélifiant est constitué de monomères styrène et d'un ou plusieurs monomères choisis parmi l'éthylène, le propylène, le butadiène, l'isoprène.

Selon un mode de réalisation de l'invention, le polymère est choisi parmi les copolymères blocs styréniques, comprenant de préférence au moins un bloc élastomère. De préférence, le copolymère bloc styrénique est choisi dans le groupe comprenant les SIS (polystyrène-polyisoprène-polystyrène), SIBS (polystyrène-polyisoprène-polybutadiène-polystyrène), SBS (polystyrène-polybutadiène-polystyrène), SEBS (polystyrène-poly(éthylènebutylène)-polystyrène) et SEPS (polystyrène-poly(éthylènepropylène)-polystyrène). De tels polymères sont par exemple disponibles auprès de la société Kraton, à travers les produits commerciaux Kraton^{®} G1702 (SEP), G1650 (SEBS), D1101 (mélange de SEBS et SEP).

Selon un mode de réalisation de l'invention, le polymère est choisi parmi les homopolymères et copolymères obtenus à partir de l'isoprène. Le polymère peut par exemple être choisi parmi les polyisoprènes. De tels polymères sont par exemple disponibles auprès de la société Kraton, à travers les produits commerciaux Kraton^{®} G 1750 (EP) ou Cariflex^{®} IR.

Selon un mode de réalisation particulier de l'invention, le polymère est choisi parmi les copolymères styrène/butadiène, les copolymères styrène/isoprène, les copolymères styrène/butadiène hydrogénés, les copolymères styrène/isoprène hydrogénés, les polymères croisés polyisoprène hydrogénés (en anglais « hydrogenated polyisoprene crosspolymer »), les homopolymères polyisoprène, les homopolymères thermoplastiques de styrène hydrogénés, les caoutchoucs liquides, et leurs mélanges.

Le polymère peut représenter de 0,5 à 50% en poids, de préférence de 1 à 40% en poids, de préférence encore de 5 à 30% en poids, du poids total de la composition gélifiée.

Selon un autre mode de réalisation de l'invention, le gélifiant est un gélifiant non-polymérique choisi de préférence parmi les charges minérales, les cires, les sels d'ammonium et les sels métalliques, de préférence encore parmi les charges minérales, les cires et les sels métalliques.

Parmi les charges minérales qui peuvent être utilisées, on peut citer les argiles, les montmorillonites, les silicates tels que les silicates d'aluminium, les silices et les hectorites. La surface des charges minérales peut être modifiée ou non modifiée, et dans le cas d'une modification de surface, la modification de surface peut être réalisée à l'aide de greffons d'origine biologique ou non biologique.

Parmi les cires (non-polymériques) qui peuvent être utilisées, on peut citer les cires naturelles telles que les cires végétales, les cires animales, les ozokérites et les cires de cérésines. Les cires végétales peuvent être choisies parmi les cires de karité, camellia, tournesol, candelilla, carnauba. Les cires animales peuvent être choisies parmi les cires d'abeille.

Parmi les sels métalliques, on peut citer le stéarate de zinc.

Selon un mode de réalisation de l'invention, le gélifiant est un gélifiant non-polymérique choisi parmi :
- Les charges minérales choisies parmi les argiles, les montmorillonites, les silicates d'aluminium, les silices et les hectorites,
- Les cires choisies parmi les cires végétales, les cires animales, les ozokérites et les cires de cérésines,
- Les sels métalliques.

Le gélifiant non-polymérique peut représenter de 0,5 à 50% en poids, de préférence de 1 à 40% en poids, de préférence encore de 5 à 30% en poids, du poids total de la composition gélifiée.

Selon un mode de réalisation de l'invention, la composition gélifiée comprenant au moins un gélifiant polymère tel que défini dans la présente invention et au moins un gélifiant non-polymérique.

La composition gélifiée selon l'invention est de préférence non aqueuse.

Selon un mode de réalisation, la composition gélifiée est de préférence substantiellement exempte d'antioxydant, autrement dit, la composition gélifiée comprend de préférence moins de 150 ppm en poids d'antioxydant, de préférence encore moins de 100 ppm en poids d'antioxydant, de préférence encore est totalement exempte d'antioxydant, l'antioxydant pouvant être choisi parmi les composés de type phénol encombré, tel que le BHT (2,6-di-tert-butyl-4-méthylphénol).

Selon un mode de réalisation de l'invention, la composition gélifiée consiste en une ou plusieurs huiles hydrocarbonées telles que définies ci-dessus et en un ou plusieurs gélifiants tels que définis ci-dessus.

Selon un mode de réalisation de l'invention, la composition gélifiée consiste en au moins une huile hydrocarbonée telle que définie ci-dessus et en au moins un polymère tel que défini ci-dessus.

L'invention porte sur une composition gélifiée telle que définie ci-dessus, de préférence à l'exception d'une composition consistant en :
- un mélange comprenant pour 100% de sa masse:
   i) 3,7% d'alcanes linéaires comportant de 15 à 19 atomes de carbone,
   ii) 96% % d'iso-alcanes comportant de 15 à 19 atomes de carbone,
   iii) 0,3 % de cyclo-alcanes comportant de 15 à 19 atomes de carbone,
- et un copolymère hydroxyethyl acrylate / acryloyldimethyltaurate acrylate copolymer (nom INCI),

le rapport massique mélange/copolymère étant de 15/2 ;
et/ou à l'exception d'une composition consistant en :
   - un mélange M'₁ comprenant pour 100% de sa masse:
      i) 3,7% d'alcanes linéaires comportant de 15 à 19 atomes de carbone,
      ii) 96% % d'iso-alcanes comportant de 15 à 19 atomes de carbone,
      iii) 0,3 % de cyclo-alcanes comportant de 15 à 19 atomes de carbone,
   - un mélange M'₂ comprenant pour 100% de sa masse:
      i) 13,20% massique d'alcanes linéaires comportant de 15 à 19 atomes de carbone,
      ii) 55,00% massique d'iso-alcanes comportant de 15 à 19 atomes de carbone,
      iii) 31,80% de cyclo-alcanes comportant de 15 à 19 atomes de carbone,
   - et un copolymère hydroxyethyl acrylate / acryloyldimethyltaurate acrylate copolymer (nom INCI),
le rapport massique mélange M'₁/mélange M'₂/copolymère étant de 12/3/2.

On peut en effet noter que le copolymère hydroxyethyl acrylate / acryloyldimethyltaurate acrylate copolymer n'est pas capable de former un gel avec une huile apolaire. Ce type de polymère pourra éventuellement former un gel avec une phase aqueuse.

### Préparation de la composition gélifiée :

La composition gélifiée peut être préparée selon tout procédé bien connu de l'homme du métier pour formuler un gel. Par exemple, la composition gélifiée selon l'invention peut être préparée par un procédé comprenant les étapes suivantes :
- Mélange de l'huile hydrocarbonée et du gélifiant, en particulier du polymère gélifiant;
- Chauffage du mélange huile hydrocarbonée et gélifiant, en particulier polymère gélifiant, de préférence à une température allant de 40°C à 180°C ou allant de 60°C à 150°C ;
- Agitation du mélange chauffé jusqu'à l'obtention d'un mélange homogène ;
- Refroidissement du mélange homogène typiquement à température ambiante (environ 25°C).

Selon un autre mode de réalisation, l'huile hydrocarbonée est préalablement chauffée avant mélange avec le gélifiant, en particulier le polymère gélifiant.

### Additifs :

La composition gélifiée selon l'invention peut également être mise en mélange avec tout adjuvant ou additif habituellement utilisé dans les domaines considérés et notamment dans les domaines cosmétique, dermatologique ou pharmaceutique. Bien entendu, l'homme du métier veillera à choisir le ou les éventuels additifs de la composition selon l'invention de manière telle que les propriétés avantageuses attachées intrinsèquement à la composition gélifiée conforme à l'invention ne soient pas ou substantiellement pas, altérées par l'addition envisagée. Parmi les adjuvants classiques susceptibles d'être contenus (selon le caractère hydrosoluble ou liposoluble de ces adjuvants), on peut citer notamment les tensioactifs moussants anioniques (tels que lauryl ether sulfate de sodium, alkyl phosphate de sodium, trideceth sulfate de sodium), amphotères (tels que alkyl bétaine, disodium cocoamphodiacetate) ou non ioniques de HLB supérieure à 10 (tels que POE/PPG/POE, Alkylpolyglucoside, polyglyceryl-3hydroxylauryl ether) ; les conservateurs tels que le chlorure de benzalkonium; les séquestrants (EDTA) ; les antioxydants ; les parfums ; les matières colorantes telles que les colorants solubles, les pigments et les nacres ; les charges matifiantes, tenseurs, blanchissantes ou exfoliantes ; les filtres solaires ; les actifs cosmétiques ou dermatologiques et agents ayant pour effet d'améliorer les propriétés cosmétiques de la peau, hydrophiles ou lipophiles ; les électrolytes. Les quantités de ces différents adjuvants sont celles classiquement utilisées dans le domaine considéré, et par exemple de 0,01 à 20 % du poids total de la composition. Comme actifs utilisables dans la composition gélifiée de l'invention, on peut citer par exemple, les vitamines hydrosolubles ou liposolubles comme la vitamine A (rétinol ou béta-carotène), la vitamine E (tocophérol), la vitamine C (acide ascorbique), la vitamine B5 (panthénol), la vitamine B3 (niacinamide), les dérivés de ces vitamines (notamment esters) et leurs mélanges ; les antiseptiques ; les actifs antibactériens comme le 2,4,4'-trichloro-2'-hydroxy diphényl éther (ou triclosan), le 3,4,4'-trichlorocarbanilide (ou triclocarban) ; les antisébohrréïques ; les antimicrobiens tels que le peroxyde de benzoyle, la niacine (vit. PP) ; les amincissants tels que la caféine ; les azurants optiques, et tout actif approprié pour le but final de la composition, et leurs mélanges.

### Composition cosmétique, dermatologique ou pharmaceutique :

La présente invention a également pour objet une composition cosmétique, dermatologique ou pharmaceutique comprenant la composition gélifiée selon l'invention et :
- au moins un corps gras choisi parmi : les huiles végétales, les beurres végétaux, les éthers et alcools gras, les esters huileux, les alcanes et les huiles silicones, de préférence parmi les esters huileux, et/ou
- au moins un additif choisi parmi les additifs précités, de préférence parmi les tensioactifs moussants anioniques (tels que lauryl éther sulfate de sodium, alkyl phosphate de sodium, tridéceth sulfate de sodium), amphotères (tels que alkyl bétaine, disodium cocoamphodiacétate) ou non ioniques de HLB supérieure à 10 (tels que POE/PPG/POE, Alkylpolyglucoside, polyglycery1-3hydroxylauryl éther).

Le corps gras est de préférence choisi parmi les corps gras présentant une faible polarité.

Des exemples d'huiles végétales sont notamment les huiles de germe de blé, de tournesol, de pépins de raisin, de sésame, de maïs, d'abricot, de ricin, de karité, d'avocat, d'olive, de soja, l'huile d'amande douce, de palme, de colza, de coton, de noisette, de macadamia, de jojoba, de luzerne, de pavot, de potimarron, de sésame, de courge, de colza, de cassis, d'onagre, de millet, d'orge, de quinoa, de seigle, de carthame, de bancoulier, de passiflore, de rosier muscat ou de camélia.

Les beurres végétaux sont des corps gras qui ont les mêmes propriétés que les huiles végétales. La différence entre les deux consiste dans le fait que les beurres se présentent sous forme solide à température ambiante. Aussi, contrairement aux huiles végétales, la matière première dont est extrait un beurre (pulpe, graines ou amandes) est chauffée après avoir été broyée pour l'extraction de la matière grasse. Comme les huiles végétales, les beurres peuvent être raffinés pour assurer une meilleure conservation, neutraliser les odeurs, améliorer la couleur et la consistance. Riches en antioxydants et nourrissants les propriétés cosmétiques des beurres végétaux améliorent l'élasticité de la peau, protègent des agressions extérieures en laissant un film protecteur sur l'épiderme et réduisant ainsi la déshydratation, réparent et apaisent en régénérant le film hydrolipidique naturel de la peau. Des exemples de beurres végétaux sont notamment le beurre de karité, le beurre de cacao, le beurre de mangue, le beurre de shorea ou encore le beurre d'olive.

Les éthers et les alcools gras sont des substances cireuses grasses à longue chaîne et aux propriétés remarquables notamment filmogènes, émollientes, hydratantes, adoucissantes et protectrices. Ils agissent comme huiles hydratantes et comme émulsifiants. Des exemples d'alcool gras ou d'éthers sont : le cetyl Alcohol, le Stearyl Alcohol, le myristyl alcohol, l'auryl alcohol, le behenyl alcohol, le cetearyl alcohol, le dicaprylyl éthers, les stearyl éthers ou l'octyldodecanol (identifiés par leur dénomination INCI).

Les esters huileux ou huiles estérifiées sont le produit d'une réaction entre des acides gras (acides à chaînes plus longues, comme par exemple l'acide stéarique, l'acide oléique, l'acide palmitique) et des alcools (des alcools gras ou des polyols comme le glycérol). Ces huiles peuvent contenir des substances issues de la pétrochimie, comme c'est le cas pour I'isopropyl Palmitate. Des exemples d'esters huileux sont le caprylic capric triglyceride, le coco caprylate caprate, l'oleyl erucate, l'oleyl linoleate, le decyl oleate ou encore le PPG-3 benzyl éther myristate (identifiés par leur dénomination INCI).

On entend par huiles de silicones ou polysiloxanes, une huile comprenant au moins un atome de silicium, et notamment au moins un groupe Si-O. Comme huile de silicone, on peut notamment citer le phenylpropyldimethylsiloxysilicate, les dimethicones ou encore le cyclopentasiloxane (identifiés par leur dénomination INCI).

Cette composition cosmétique, dermatologique ou pharmaceutique comprend un milieu physiologiquement acceptable, c'est-à-dire qui ne présente pas d'effets secondaires délétères et en particulier qui ne produit pas de rougeurs, d'échauffements, de tiraillements ou de picotements inacceptables pour un utilisateur. Ce milieu comprend éventuellement de l'eau et/ou au moins une huile en tant que corps gras, en plus de la composition gélifiée précitée.

Selon un mode de réalisation la composition cosmétique, dermatologique ou pharmaceutique a une teneur en composition gélifiée telle que décrite ci-dessus allant de 0,5 à 80%, de préférence de 1 à 50% et avantageusement de 5 à 30% en poids par rapport au poids total de la composition.

De préférence, lorsque le corps gras est choisi parmi les huiles végétales, le corps gras représentera de préférence moins de 50% en poids, de préférence encore moins de 30% en poids, du poids de la composition cosmétique, dermatologique ou pharmaceutique.

De préférence, lorsque le corps gras est choisi parmi les huiles de silicones ou polysiloxanes, le corps gras représentera de préférence moins de 40% en poids, de préférence encore moins de 30% en poids, du poids de la composition cosmétique, dermatologique ou pharmaceutique.

La composition cosmétique, dermatologique ou pharmaceutique selon l'invention peut ainsi être une composition anhydre, une émulsion telle qu'une émulsion eau-dans-huile (E/H), une émulsion huile-dans-eau (H/E) ou une émulsion multiple (notamment E/H/E ou H/E/H), une nanoémulsion, ou encore une dispersion, selon les additifs éventuellement introduits et/ou selon une phase aqueuse éventuellement introduite.

Lorsque la composition est une émulsion de type huile-dans-eau, de préférence, l'invention ne couvre pas une émulsion à usage topique de type huile-dans-eau (E) comprenant pour 100% de sa masse:
- De 50% à 90% massique, d'une phase aqueuse (A) cosmétiquement acceptable,
- De 10% à 50% massique, d'une phase grasse (G) comprenant pour 100% de sa masse :
   - De 10% à 50% massique, plus particulièrement de 15% à 40% massique d'un mélange (M1) d'hydrocarbures saturés cycliques ou acycliques, linéaires ou ramifiés parmi lesquels au moins 95% massique comporte de quinze à dix-neuf atomes de carbone;
   - De 0,5% à 15% massique, d'au moins un agent tensioactif de type huile-dans-eau,
   - De 5% à 30% massique, d'au moins un agent de protection contre les rayonnements ultra-violets du soleil,
- De 0% à 80% massique, d'au moins une huile et/ou une cire, étant entendu qu'une telle huile et/ou une telle cire ne répond pas à la définition du mélange (M1) ;

Lorsque la composition est une émulsion de type huile-dans-eau, de préférence, l'invention ne couvre pas une émulsion de type huile-dans-eau (E) comprenant pour 100% de sa masse:
- de 40% à 90%, massique d'une phase aqueuse (A) cosmétiquement acceptable comprenant pour 100% de sa masse de 1% massique à 30% massique de glycérol, et
- de 10% à 60% massique d'une phase grasse (G) comprenant pour 100% de sa masse de 1% à 25% massique d'un mélange (M1) d'hydrocarbures saturés cycliques ou acycliques, linéaires ou ramifiés parmi lesquels au moins 95% massique comporte de quinze à dix-neuf atomes de carbone et de 0,5% à 15 % d'au moins un agent tensioactif de type huile-dans-eau.

La composition cosmétique, dermatologique ou pharmaceutique selon l'invention peut constituer par exemple une composition de démaquillage ou de nettoyage de la peau, des lèvres, une composition solaire (protection U.V.), ou après-solaire, une composition pour le massage de la peau, une composition de baume soin douche, une composition anti-transpirante, une composition de masque, une composition de baume réparateur, une composition gommante et/ou exfoliante aussi bien pour le visage que pour les mains (lorsqu'elle contient des particules exfoliantes), une composition de maquillage, une composition de rasage, une composition de baume après-rasage, une composition parfumée, une composition pour lingettes.

La composition de l'invention se caractérise avantageusement par le fait qu'elle présente une stabilité d'une durée supérieure ou égale à 4 semaines, avantageusement supérieure ou égale à 6 semaines, la stabilité étant évaluée après un stockage sans agitation à température ambiante, à 40°C et à 50°C et correspondant à une évaluation visuelle de la coloration et de l'aspect ainsi qu'une évaluation olfactive et/ou une mesure de la viscosité.

### Utilisation de la composition gélifiée :

L'invention a encore pour objet l'utilisation cosmétique, dermatologique ou pharmaceutique de la composition telle que définie ci-dessus pour une application topique, sur la peau, les lèvres ou les phanères (incluant les ongles, le cuir chevelu et les cheveux).

L'invention a aussi pour objet l'utilisation cosmétique, dermatologique ou pharmaceutique de la composition cosmétique, dermatologique ou pharmaceutique telle que définie ci-dessus comme produit de soin de la peau (sérums, crèmes, baumes, etc), comme produit d'hygiène, comme produit solaire/après-solaire, comme produit de maquillage, comme produit démaquillant, comme produit parfumé, comme produit anti-transpirant, comme produit de soin des lèvres, tel que le gloss ou les sticks hydratants pour les lèvres.

La composition gélifiée selon l'invention peut être utilisée, de préférence comme principe actif, pour améliorer l'aspect de la peau et/ou pour tonifier les cheveux et/ou pour ralentir le vieillissement et/ou pour retarder l'apparition des rides et/ou pour diminuer les rides et/ou pour tendre la peau. En particulier, les inventeurs ont découvert que la composition gélifiée présentait un effet antioxydant ainsi qu'un effet antiradicalaire.

La composition gélifiée selon l'invention peut être utilisée, de préférence comme principe actif, comme médicament.

Selon un aspect de l'invention, la composition gélifiée peut être utilisée en tant qu'agent antioxydant et/ou agent antiradicalaire et/ou agent anti-inflammatoire et/ou anti-apoptotique et/ou antibactérien et/ou antifongique.

L'invention a encore pour objet un procédé cosmétique, dermatologique ou pharmaceutique de traitement de la peau, comprenant au moins une étape d'application sur la peau, les lèvres et/ou les phanères d'une composition telle que définie ci-dessus.

La composition de l'invention peut également être utilisée pour la formulation de compositions cosmétiques, de compositions dermatologiques ou de compositions pharmaceutiques comprenant d'autres composants ou d'autres phases que ceux décrits ci-dessus. Il peut s'agir notamment de la formulation de compositions de soin, d'hygiène, de maquillage.

### Procédé de traitement cosmétique :

Enfin, l'invention couvre aussi un procédé de traitement cosmétique comprenant au moins une étape d'application, de préférence par étalement, sur la peau, les lèvres ou les phanères, des compositions selon l'invention.

### EXEMPLES

Dans la suite de la présente description, des exemples sont donnés à titre illustratif de la présente invention et ne visent en aucun cas à en limiter la portée.

### Composition gélifiée :

Une composition gélifiée comprenant 90% en poids d'une huile hydrocarbonée et 10% en poids d'un copolymère dibloc linéaire à base de styrène et d'éthylène/propylène avec une teneur en polystyrène de 28% en poids/poids du polymère. La composition gélifiée a été préparée selon un procédé tel que décrit dans la présente invention.

Le tableau 1 regroupe les propriétés physico chimiques de l'huile hydrocarbonée.

**Tableau 1 : propriétés physico chimiques de l'huile hydrocarbonée**

| Caractéristiques | Huile hydrocarbonée |
|---|---|
| Aromatiques (ppm) | <20 |
| Soufre (ppm) | 0,11 |
| % iso paraffines (w/w) | 96,2 |
| % n-paraffines (w/w) | 3,8 |
| % naphténiques (w/w) | 0 |
| C13 (iso) | 0 |
| C14 (iso) | 0 |
| C15 (iso) | 0 |
| C16 (iso) | 1,58 |
| C17 (iso) | 14,17 |
| C18 (iso) | 79,69 |
| C19 (iso) | 0,12 |
| C20 (iso) | 0,38 |
| C27 (iso) | 0,29 |
| Quantité de carbones d'origine biologique (%) | > 98 |
| Point d'ébullition initial (°C) | 293,6 |
| Point ébullition 5% (°C) | 296,7 |
| Point ébullition 50% (°C) | 298,5 |
| Point ébullition 95% (°C) | 305,3 |
| Point d'ébullition final (°C) | 324,1 |
| Biodégradabilité OECD (28 jours) (%) | 83 |
| Indice de réfraction à 20°C | 1,4394 |
| densité à 15°C (kg/m3) | 787,2 |
| Point éclair (°C) | 149 |
| Viscosité Cinématique à 40°C (cSt) | 3,87 |
| Pression de vapeur à 20°C (kPa) | <0,01 |
| Point Aniline (°C) | 93,2 |

Les normes et méthodes suivantes ont été utilisées pour mesurer les propriétés ci-dessus :
- point éclair : EN ISO 2719
- densité à 15°C : EN ISO 1185
- viscosité à 40°C : EN ISO 3104
- point aniline : EN ISO 2977
- Point d'ébullition : ASTM D86
- biodégradabilité : méthode OCDE 306
- indice de réfraction à 20°C : ASTM D 1218
- pression de vapeur : calculée selon des méthodes bien connues de l'homme du métier

### Evaluation de la miscibilité de la composition gélifiée avec des corps gras

Les corps gras testés sont :
- une huile végétale : meadowfoam seed oil (selon la dénomination INCI) présentant une densité à 20°C de 0,91 et comprenant :
   ∘ 58,0-64,0% en poids d'acide gadoléique (C20 :1)
   ∘ 10,0-14,0% en poids d'acide érucique (C22:1 d13)
   ∘ 3,0-6,0% en poids d'acide docosénoïque (C22:1 d5)
   ∘ 15,0-21,0% en poids d'acide docosadiénoïque (C22:2)
   Par rapport au poids total de l'huile végétale.
- Un ester huileux : isononyl isononanoate (selon la dénomination INCI), disponible commercialement.
- Une huile silicone : cyclopentasiloxane (selon la dénomination INCI), disponible commercialement.

La manipulation consiste à observer la miscibilité de la composition gélifiée avec les corps gras, en procédant à l'ajout du corps gras à tester dans la composition gélifiée par pourcentage en poids croissant : 10%, 25%, 50%, 75% et 90%. Ces manipulations sont réalisées en continu. On observe le mélange entre chaque ajout. L'ajout est effectué dans le même récipient.

Les expériences sont réalisées sur une base de départ de 45g de composition gélifiée pour finir à 450g. En effet, les ajouts sont effectués comme tel sur la base de 45g, présente dans le bécher :
- 10% : ajout de 5g de corps gras à tester.
- 25% : ajout de 10g de corps gras à tester dans le mélange précédemment obtenu
- (15*100/60=25).
- 50% : ajout de 30g de corps gras à tester dans le mélange précédemment obtenu.
- 75% : ajout de 90g de corps gras à tester dans le mélange précédemment obtenu.
- 90% : ajout de 270g de corps gras à tester dans le mélange précédemment obtenu.

Les mélanges finaux sont conservés dans des pots en verre et observés à J+1. Un mélange est dit non miscible lorsque des sédiments sont visuellement observés.

Il a ainsi été observé que :
- La composition gélifiée selon l'invention est miscible avec l'huile végétale, lorsque l'huile végétale est présente en une quantité allant jusqu'à 50% en poids.
- La composition gélifiée selon l'invention est miscible avec l'ester huileux, lorsque l'ester huileux est présent en une quantité allant jusqu'à 90% en poids.
- La composition gélifiée selon l'invention est miscible avec l'huile silicone, lorsque l'huile silicone est présente en une quantité allant jusqu'à 25% en poids.

### Evaluation de la compatibilité de la composition gélifiée avec des émulsionnants

### Préparation des émulsions :

Les émulsionnants huile-dans-eau (H/E) suivants ont été testés :
- Un émulsionnant anionique : Sensanov WR^{®} (disponible auprès de Seppic), correspondant au produit C20-22 Alkyl Phosphate & C20-22 Alcohols (selon la dénomination INCI).
- Deux émulsionnants non-ioniques :
   ∘ Montanov^{®} 68 (disponible auprès de Seppic), correspondant à Cetearyl Alcohol (and) Cetearyl Glucoside (selon la dénomination INCI).
   ∘ Simulsol^{®} 165 (disponible auprès de Seppic), correspondant au produit PEG-100 Stearate (and) Glyceryl Stearate (selon la dénomination INCI).
- Un émulsionnant cationique : Incronat^{®} Behenyl TMS (disponible auprès de Croda), correspondant au produit Cetearyl Alcohol, Behentrimonium Methosulfate (selon la dénomination INCI).

Un émulsionnant eau-dans-huile (E/H) a également été testé :
- Emulsionnant non-ionique : Abil^{®} EM180 (disponible auprès de Evonik), correspondant au produit Cetyl PEG/PPG-10/1 Diméthicone (selon la dénomination INCI).

Des émulsions 1 à 5 ont été préparées à partir des 5 émulsionnants décrits ci-dessus. Le tableau 2 ci-dessous indique la composition des émulsions 1 à 5 testées.

Les émulsions (H/E) 1 à 4 ont été préparées selon le mode opératoire suivant :
- L'eau est pesée, mise sous agitation et chauffée à 50°C. A 50°C, le prémix Glycérine et Gomme Xanthane est versé sous vive agitation. L'agitation est maintenue jusqu'à l'obtention d'une phase homogène. Enfin la chauffe est poursuivie à 75-80°C (selon l'émulsionnant).
- La phase grasse est pesée, mise sous agitation et chauffée à 75-80°C.
- A 75-80°C, la phase grasse est versée progressivement dans la phase aqueuse sous vive agitation. Le refroidissement est alors amorcé.
- A 72-75°C l'émulsion est agitée à l'aide d'un appareil de type Turrax^{®} 30 secondes à 9500rpm.
- Le refroidissement est ensuite poursuivi.
- A 50°C, le conservateur est versé dans l'émulsion sous agitation.
- A 25°C, le pH est mesuré et ajusté si nécessaire (soit avec une solution de soude à 10%, soit
- avec une solution d'acide citrique à 50%) : la fourchette souhaitée se situant entre 5,00 et 7,00.

L'émulsion (E/H) 5 a été préparée selon le mode opératoire suivant :
- L'eau et la glycérine sont pesées, mises sous agitation. Le sel, puis le conservateur y sont ajoutés.
- L'émulsionnant est pesé, mis sous agitation. L'ester y est ajouté sous agitation, puis la composition gélifiée
- Lorsque les deux phases sont homogènes : la phase aqueuse est ajoutée lentement dans la phase grasse, au goutte à goutte.
- L'ajout peut être accéléré dès que plus d'un tiers de la phase aqueuse est ajoutée.

**Tableau 2 : Description des émulsions testées 1 à 5 (pourcentages exprimés en poids par rapport au poids total de l'émulsion)**

| | | Emulsion 1 | Emulsion 2 | Emulsion 3 | Emulsion 4 | Emulsion 5 |
|---|---|---|---|---|---|---|
| Phase grasse | Sensanov WR^{®} | 3,00% | | | | |
| | Montanov^{®} 68 | | 5,00% | | | |
| | Simulsol^{®} 165 | | | 5,00% | | |
| | Incronat^{®} Behenyl TMS | | | | 5,00% | |
| | Abil^{®} EM180 | | | | | 2,00% |
| | Cetearyl Alcohol | 2,00% | 2,00% | 2,00% | 2,00% | |
| | Composition gélifiée | 5,00% | 5,00% | 5,00% | 5,00% | 5,00% |
| | Ester (isononyl Isononanoate) | 10,00% | 10,00% | 10,00% | 10,00% | 10,00% |
| Phase aqueuse | Eau déminéralisée | Qsp 100% | Qsp 100% | Qsp 100% | Qsp 100% | Qsp 100% |
| | Glycérine | 1,00% | 1,00% | 1,00% | 1,00% | 1,00% |
| | Gomme xanthane | 0,25% | 0,25% | 0,25% | | |
| | conservateur (méthylisothiazolinone) | 0,10% | 0,10% | 0,10% | 0,10% | 0,10% |
| | NaCl (sel) | | | | | 0,50% |
| Température pour la préparation de l'émulsion | | 80°C⁽¹⁾ | 75°C | 75°C | 80°C⁽¹⁾ | 23°C |
| Viscosité à 25°C à J+1 (mPa.s) | | 16500 | 5500 | 9000 | 9000 | 120000 |

| | | | | | | |
|---|---|---|---|---|---|---|
| (1) Le pH a été ajusté à l'aide d'une solution de soude à 10% | | | | | | |

### Observation au microscope de chaque émulsion :

La compatibilité de la composition gélifiée selon l'invention avec les émulsionnants a été évaluée visuellement par une observation au microscope de chaque émulsion.

### Stabilité de la viscosité au cours du temps :

La viscosité des émulsions a également été mesurée à l'aide du Brookfield RV DVII+PRO pendant 1 minute dans des pots de 200g ayant été préalablement thermostatés entre 24,5 et 25,5°C. En particulier, l'évolution de la viscosité au cours du temps a été évaluée : à J0, J+1 (24 heures), 2 semaines, 4 semaines et 8 semaines. Une note « + » signifie une stabilité satisfaisante et une note « ++ » signifie une très bonne stabilité.

### Stabilité du pH au cours du temps :

Le pH est également mesuré au cours du temps de J0 (initialement) à 8 semaines. Une note « + » signifie une stabilité satisfaisante avec une tendance à la baisse au cours du temps et en température, une note « ++ » signifie une bonne stabilité avec une légère tendance à la baisse en température et une note « +++ » signifie une très bonne stabilité.

### Stabilité à la centrifugation :

Une centrifugation est mise en œuvre sur les émulsions à J+1 (24 heures), pendant 1h30 à 4000rpm.

Une note « + » signifie une stabilité satisfaisante avec formation d'un petit culot d'eau et une note « ++ » signifie une très bonne stabilité sans aucune instabilité.

Les résultats de ces tests sont rassemblés dans le tableau 3 ci-dessous.

**Tableau 3 : évaluation des propriétés des émulsions 1 à 5**

| | Emulsion 1 | Emulsion 2 | Emulsion 3 | Emulsion 4 | Emulsion 5 |
|---|---|---|---|---|---|
| Observation au microscope | Tapis de gouttelettes grossières, peu dense et homogène | Tapis de gouttelettes moyennement fin, très dense et homogène | Tapis de gouttelettes relativement fin, dense et homogène | Tapis de gouttelettes moyennement fin, moyennement dense et homogène | Tapis de gouttelettes très fin, très dense et homogène |
| Stabilité de la viscosité | ++ | + | ++⁽¹⁾ | ++⁽¹⁾ | ++ |
| Stabilité du pH | + | +++ | + | ++ | (2) |
| Stabilité (centrifugation) | ++ | ++ | ++ | + | ++ |

| | | | | | |
|---|---|---|---|---|---|
| (1) à partir de J+1 (2) Non applicable à ce type d'émulsion (E/H) | | | | | |

En conclusion, on peut noter que la composition gélifiée selon l'invention est compatible avec différents types d'émulsionnants.

### Préparation de formulations cosmétiques comprenant la composition gélifiée

### - Crème pour le visage

Une crème pour le visage présentant la composition indiquée dans le tableau 4 ci-dessous a été préparée. La teneur de chaque ingrédient est indiquée en pourcentage en poids par rapport au poids total de la composition de crème. Pour les ingrédients différents de la composition gélifiée préparée précédemment, le nom INCI (UE) est donné.

**Tableau 4 : composition de la crème pour le visage**

| Phase de préparation | Nom commercial | ingrédients | Teneur en % en poids |
|---|---|---|---|
| | MONTANOV^{®} 68 | CETEARYL ALCOHOL | 5,00 |
| | | CETEARYL GLUCOSIDE | |
| | | GLUCOSE | |
| | | AQUA | |
| A | CUTINA^{®} PES | PENTAERYTHRITYL | 3,00 |
| | | DISTEARATE | |
| | JEENATE^{®} 2H | POLYETHYLENE | 2,00 |
| | KARITE^{®} CP | BUTYROSPERMUM PARKII | 2,00 |
| | | BUTTER | |
| | MASSOCARE^{®} TH | TRIETHYLHEXANOIN | 5,00 |
| | LANOL^{®} 99 | ISONONYL ISONONANOATE | 4,00 |
| | | Composition gélifiée | 5,00 |
| B | EAU DEMINERALISEE | AQUA | 68,12 |
| | EDETA^{®} BD | DISODIUM EDTA | 0,10 |
| | | AQUA | |
| | GLYCERINE CODEX^{®} | GLYCERIN | 2,50 |
| | RHODICARE^{®} T | XANTHAN GUM | 0,30 |
| | | AQUA | |
| | CHLORPHENESIN^{®} BP 73 | CHLORPHENESIN | 0,25 |
| | | AQUA | |
| C | PHENOXETOL^{®} | PHENOXYETHANOL | 0,80 |
| | SEPINOV^{®} EMT10 | HYDROXYETHYL | 0,80 |
| | | ACRYLATE/SODIUM | |
| | | ACRYLOYLDIMETHYL | |
| | | TAURATE COPOLYMER | |
| | | SORBITAN ISOSTEARATE | |
| | | POLYSORBATE 60 | |
| | | AQUA | |
| D | JUVENESSENCE^{®} | CAPRYLIC/CAPRIC | 1,00 |
| | | TRIGLYCERIDE | |
| | | ALGAE EXTRACT | |
| | NORA^{®} SA FML00190 | PARFUM | 0,13 |

Procédé de préparation de la crème pour le visage:
a) Mettre sous agitation les ingrédients de la phase A et chauffer à 75°C jusqu'à l'obtention d'une phase homogène.
b) Peser l'eau et l'edeta BD. Mettre sous agitation et commencer la chauffe. A 50°C introduire le prémix glycerine + rhodicare T. Poursuivre l'agitation jusqu'à l'obtention d'une phase homogène, tout en poursuivant la chauffe à 75°C. A 75°C introduire la chlorphenesine. Puis à 75°C, sous 1200rpm, réaliser l'émulsion en versant la phase A (obtenue à l'étape a) dans la phase B (obtenue à l'étape b)). Commencer le refroidissement en maintenant la vitesse d'agitation. A 72°C turraxer 30s à 9500rpm puis poursuivre le refroidissement sous agitation modérée.
c) A 50°C introduire le phenoxetol, puis saupoudrer le sepinov EMT10. Maintenir sous vive agitation jusqu'à l'obtention d'un produit homogène. Puis poursuivre le refroidissement sous agitation modérée.
d) A température ambiante (23°C) ajouter successivement les composants de la phase D. Mesurer le pH et l'ajuster si nécessaire avec une solution de soude à 10% pour obtenir un pH entre 5,50 et 6,00.

### - Lait corporel hydratant

Un lait corporel hydratant présentant la composition indiquée dans le tableau 5 ci-dessous a été préparé. La teneur de chaque ingrédient est indiquée en pourcentage en poids par rapport au poids total de la composition de crème. Pour les ingrédients différents de la composition gélifiée préparée précédemment, le nom INCI (UE) est donné.

**Tableau 5 : composition du lait corporel**

| Phase de préparation | Nom commercial | ingrédients | Teneur en % en poids |
|---|---|---|---|
| A | MONTANOV^{®} L | C14-22 ALCOHOLS | 3,00 |
| | | C12-20 ALKYL GLUCOSIDE | |
| | TEGO ALKANOL^{®} 1618 | CETEARYL ALCOHOL | 1,00 |
| | TEGO ALKANOL^{®} 16 | CETYL ALCOHOL | 1,00 |
| | LANOL^{®} 99 | ISONONYL ISONONANOATE | 5,00 |
| | DUB^{®} IPP | ISOPROPYL PALMITATE | 4,00 |
| | | Composition gélifiée | 2,00 |
| | EAU DEMINERALISEE | AQUA | 74,25 |
| | EDETA^{®} BD | DISODIUM EDTA | 0,10 |
| | | AQUA | |
| | ZEMEA^{®} | PROPANEDIOL | 3,00 |
| | PROPANEDIOL | AQUA | |
| | RHODICARE^{®} T | XANTHAN GUM | 0,30 |
| | | AQUA | |
| | | HYDROXYETHYL | |
| | | ACRYLATE/SODIUM | |
| B | | ACRYLOYLDIMETHYL | |
| | | TAURATE COPOLYMER | |
| | SEPIPLUS^{®} S | POLYISOBUTENE | 0,50 |
| | | PEG-7 | |
| | | TRIMETHYLOLPROPANE | |
| | | COCONUT ETHER | |
| | | AQUA | |
| | | SORBITAN ISOSTEARATE | |
| | CHLORPHENESIN^{®} BP 73 | CHLORPHENESIN | 0,25 |
| | | AQUA | |
| C | PHENOXETOL^{®} | PHENOXYETHANOL | 0,80 |
| | MIRASIL^{®} CM5 | CYCLOPENTASILOXANE | 1,00 |
| | AQUAXYL^{®} | XYLITYLGLUCOSIDE | 3,00 |
| | | ANHYDROXYLITOL | |
| | | AQUA | |
| | | XYLITOL | |
| D | | GLUCOSE | |
| | BERYL^{®} SA FML00314 | PARFUM | 0,80 |
| | | MENTHOL | |
| | | GERANIOL | |
| | | BHT | |
| | | TOCOPHEROL | |

Procédé de préparation du lait corporel :
a) Mettre les composés de la phase A sous agitation et chauffer à 80°C jusqu'à l'obtention d'une phase homogène.
b) Mettre l'EDTA et l'eau de la phase B sous agitation jusqu'à l'obtention d'une phase homogène, tout en chauffant à 50°C. A 50°C, introduire un prémix du zemea et de rhodicare T et poursuivre la chauffe et l'agitation jusqu'à 80°C tout en vérifiant l'obtention d'une phase homogène. A 80°C, sous vive agitation, introduire le sepiplus S et maintenir sous agitation jusqu'à l'obtention d'une phase homogène. A 80°C, sous 1200rpm, introduire la phase A (obtenue à l'étape a)) dans la phase B (obtenue à l'étape b)) puis commencer le refroidissement en maintenant la vitesse d'agitation. A 72°C saupoudrer la chlorphenesine puis turraxer 30s à 9500rpm. Poursuivre le refroidissement sous agitation modérée.
c) A 50°C introduire successivement les composants de la phase C puis poursuivre le refroidissement sous agitation modérée.
e) A 35°C, introduire successivement les composants de la phase D en vérifiant la bonne homogénéité entre chaque ajout. Mesurer le pH et l'ajuster si nécessaire pour obtenir un pH entre 5,50 et 6,00.

## Revendications

1. Composition gélifiée comprenant :
- au moins 50% en poids d'au moins une huile hydrocarbonée qui comprend une teneur en poids d'isoparaffines allant de 90 à 100%, une teneur en poids de paraffines normales allant de 0 à 10% et une teneur en carbone d'origine biologique supérieure ou égale à 90 % par rapport au poids total de l'huile hydrocarbonée, ladite huile hydrocarbonée ayant une biodégradabilité à 28 jours d'au moins 60%, mesurée selon la norme OECD 306, et
- au moins 0,5% en poids d'au moins un gélifiant choisi parmi :
i. les polymères gélifiants choisis parmi les homopolymères ou les copolymères comprenant au moins un monomère choisi parmi l'isoprène, le butadiène, le styrène et les (méth)acrylates,
ii. les gélifiants non-polymériques,
par rapport au poids total de la composition gélifiée.

2. Composition gélifiée selon la revendication 1, dans laquelle le gélifiant est un polymère gélifiant choisi parmi les homopolymères ou les copolymères comprenant au moins un monomère choisi parmi l'isoprène, le butadiène, le styrène et les (méth)acrylates, de préférence le polymère gélifiant est choisi parmi les homopolymères ou les copolymères comprenant au moins un monomère choisi parmi l'isoprène, le butadiène, le styrène, encore plus préférentiellement le polymère est un copolymère comprenant au moins un monomère styrène et au moins un monomère choisi parmi l'éthylène, le propylène, le butadiène, l'isoprène.

3. Composition gélifiée selon la revendication 2, dans laquelle le polymère est choisi parmi les polymères en étoile, les copolymères diblocs, les copolymères triblocs, les copolymères multiblocs, les polymères en peigne, les polymères radiaux, et des combinaisons de ceux-ci.

4. Composition gélifiée selon l'une quelconque des revendications 2 à 3, dans laquelle le polymère est choisi parmi les copolymères styrène/butadiène, les copolymères styrène/isoprène, les copolymères styrène/butadiène hydrogénés, les copolymères styrène/isoprène hydrogénés, les polymères croisés polyisoprène hydrogénés, les homopolymères polyisoprène, les homopolymères thermoplastiques de styrène hydrogénés, les caoutchoucs liquides, et leurs mélanges.

5. Composition gélifiée selon la revendication 1, dans laquelle le gélifiant est un gélifiant non-polymérique choisi parmi:
- les charges minérales choisies de préférence parmi les argiles, les montmorillonites, les silicates, les silices, les hectorites,
- les cires choisies de préférence parmi les cires naturelles, telles que les cires végétales, les cires animales, les ozokérites et les cérésines, et
- les sels d'ammonium et les sels métalliques choisis de préférence parmi le stéarate de zinc.

6. Composition gélifiée selon l'une quelconque des revendications précédentes dans laquelle l'huile hydrocarbonée:
- est choisie parmi les isoparaffines non-cycliques comprenant de 14 à 18 atomes de carbone ; et/ou
- comprend une teneur en poids d'isoparaffines allant de 95 à 100% et préférentiellement de 98% à 100% par rapport au poids total de l'huile hydrocarbonée ; et/ou
- comprend une teneur en carbone d'origine biologique supérieure ou égale à 95%, de préférence supérieure ou égale à 98% et préférentiellement de 100% ; et/ou
- comprend une teneur en poids de paraffines normales inférieure ou égale à 5% et préférentiellement inférieure ou égale à 2% par rapport au poids total de l'huile hydrocarbonée ; et/ou
- comprend une teneur en poids de composés naphténiques inférieure ou égale à 1%, de préférence inférieure ou égale à 0,5% et préférentiellement inférieure ou égale à 100 ppm par rapport au poids total de l'huile hydrocarbonée ; et/ou
- comprend une teneur en poids de composés aromatiques inférieure ou égale à 500 ppm, de préférence inférieure ou égale à 300 ppm, préférentiellement inférieure ou égale à 100 ppm, plus préférentiellement inférieure ou égale à 50 ppm et avantageusement inférieure ou égale à 20 ppm, par rapport au poids total de l'huile hydrocarbonée ; et/ou
- a une température d'ébullition allant de 230 à 340°C, de préférence de 235 à 330°C et plus préférentiellement de 240 à 325°C selon la norme ASTM D86 ; et/ou
- a une biodégradabilité à 28 jours d'au moins 70%, préférentiellement d'au moins 75% et encore plus préférentiellement d'au moins 80% mesurée selon la norme OECD 306 ; et/ou
- a un point éclair supérieur ou égal à 110°C selon la norme EN ISO 2719 ; et/ou
- est obtenue par un procédé d'hydrogénation catalytique à une température de 80 à 180°C et à une pression de 50 à 160 bars d'une charge d'origine biologique désoxygénée et/ou isomérisée.

7. Composition gélifiée selon l'une quelconque des revendications précédentes comprenant, par rapport au poids total de la composition gélifiée, de 50 à 99% en poids, de préférence de 60 à 95% en poids, de préférence encore de 70 à 90% en poids, d'huile hydrocarbonée et de 1 à 50% en poids, de préférence de 5 à 40% en poids, de préférence encore de 10 à 30% en poids, dudit gélifiant, de préférence choisi parmi lesdits polymères gélifiants.

8. Composition cosmétique, dermatologique ou pharmaceutique comprenant au moins une composition gélifiée selon l'une des revendications 1 à 7, de préférence en une quantité allant de 0,5 à 80%, préférentiellement de 1 à 50% et avantageusement de 5 à 30% en poids par rapport au poids total de la composition.

9. Composition cosmétique, dermatologique ou pharmaceutique selon la revendication 8, comprenant au moins un corps gras choisi parmi : les huiles végétales, les huiles hydrocarbonées autres que l'huile hydrocarbonée de ladite composition gélifiée, les beurres végétaux, les éthers et alcools gras, les esters huileux, les alcanes et les huiles silicones et/ou au moins un additif de préférence choisi parmi les émulsionnants.

10. Utilisation de la composition gélifiée selon l'une quelconque des revendications 1 à 7, ou de la composition selon la revendication 8 ou 9 pour une application topique, notamment comme produit de soin pour la peau ou des cheveux, comme produit de maquillage, comme produit capillaire, comme produit démaquillant, comme produit de soin des lèvres, tel que le gloss ou les sticks hydratants pour les lèvres.

11. Procédé de traitement cosmétique de la peau comprenant au moins une étape d'application, de préférence par étalement, de la composition gélifiée selon l'une quelconque des revendications 1 à 7 ou de la composition selon la revendication 8 ou 10.

## Patentansprüche

1. Gelierte Zusammensetzung, umfassend:
- mindestens 50 Gewichts-% mindestens eines Kohlenwasserstofföls, das einen Gewichtsgehalt an Isoparaffinen von 90 bis 100 %, einen Gewichtsgehalt an normalen Paraffinen von 0 bis 10 % und einen Gehalt an Kohlenstoff biologischen Ursprungs von 90 % oder mehr, bezogen auf das Gesamtgewicht des Kohlenwasserstofföls, umfasst, wobei das Kohlenwasserstofföl eine biologische Abbaubarkeit nach 28 Tagen von mindestens 60 %, gemessen nach der Norm OECD 306, aufweist, und
- mindestens 0,5 Gewichts-% mindestens eines Geliermittels, ausgewählt aus:
i. gelbildenden Polymeren, die ausgewählt sind aus Homopolymeren oder Copolymeren, umfassend mindestens ein Monomer, das ausgewählt ist aus Isopren, Butadien, Styrol und (Meth)acrylaten,
ii. nicht-polymere Geliermittel,
bezogen auf das Gesamtgewicht der gelierten Zusammensetzung.

2. Gelierte Zusammensetzung nach Anspruch 1, wobei das Geliermittel ein gelbildendes Polymer ist, das ausgewählt ist aus Homopolymeren oder Copolymeren, umfassend mindestens ein Monomer, das ausgewählt ist aus Isopren, Butadien, Styrol und (Meth)acrylaten, vorzugsweise das gelbildende Polymer ausgewählt ist aus Homopolymeren oder Copolymeren, umfassend mindestens ein Monomer, das ausgewählt ist aus Isopren, Butadien, und Styrol, noch bevorzugter das Polymer ein Copolymer ist, umfassend mindestens ein Styrolmonomer und mindestens ein Monomer, das ausgewählt ist aus Ethylen, Propylen, Butadien und Isopren.

3. Gelierte Zusammensetzung nach Anspruch 2, wobei das Polymer ausgewählt ist aus Sternpolymeren, Zweiblock-Copolymeren, Dreiblock-Copolymeren, Mehrblock-Copolymeren, Kamm-Polymeren, Radial-Polymeren und Kombinationen davon.

4. Gelierte Zusammensetzung nach einem der Ansprüche 2 bis 3, wobei das Polymer ausgewählt ist aus Styrol/Butadien-Copolymeren, Styrol/Isopren-Copolymeren, hydrierten Styrol/Butadien-Copolymeren, hydrierten Styrol/Isopren-Copolymeren, hydrierten Polyisopren-Kreuzpolymeren, Polyisopren-Homopolymeren, hydrierten thermoplastischen Styrol-Homopolymeren, Flüssigkautschuk und deren Gemischen.

5. Gelierte Zusammensetzung nach Anspruch 1, wobei das Geliermittel ein nicht-polymeres Geliermittel ist, das ausgewählt ist aus:
- mineralischen Füllstoffen, die vorzugsweise ausgewählt sind aus Ton, Montmorillonit, Silikaten, Kieselsäure, Hectorit,
- Wachsen, die vorzugsweise ausgewählt sind aus natürlichen Wachsen, wie beispielsweise Pflanzenwachsen, tierischen Wachsen, Ozokeriten und Ceresinen, und
- Ammoniumsalzen und Metallsalzen, die vorzugsweise ausgewählt sind aus Zinkstearat ausgewählt.

6. Gelierte Zusammensetzung nach einem der vorhergehenden Ansprüche, wobei das Kohlenwasserstofföl:
- ausgewählt ist aus nicht-zyklischen Isoparaffinen, umfassend 14 bis 18 Kohlenstoffatome; und/oder
- einen Isoparaffingehalt von 95 bis 100 Gewichts-% und vorzugsweise 98 bis 100 Gewichts-%, bezogen auf das Gesamtgewicht des Kohlenwasserstofföls, umfasst; und/oder
- einen Gehalt an Kohlenstoff biologischen Ursprungs von mehr als oder gleich wie 95 %, vorzugsweise mehr als oder gleich wie 98 % und bevorzugt 100 % aufweist; und/oder
- einen Gewichtsgehalt an normalen Paraffinen von weniger als oder gleich wie 5 % und vorzugsweise weniger als oder gleich wie 2 %, bezogen auf das Gesamtgewicht des Kohlenwasserstofföls, umfasst; und/oder
- einen Gewichtsgehalt an naphthenischen Verbindungen von weniger als oder gleich wie 1 %, vorzugsweise weniger als oder gleich wie 0,5 % und besonders bevorzugt weniger als oder gleich wie 100 ppm, bezogen auf das Gesamtgewicht des Kohlenwasserstofföls, umfasst; und/oder
- einen Gewichtsgehalt an aromatischen Verbindungen von weniger als oder gleich wie 500 ppm, vorzugsweise weniger als oder gleich wie 300 ppm, bevorzugt weniger als oder gleich wie 100 ppm, noch bevorzugter weniger als oder gleich wie 50 ppm und vorteilhafterweise weniger als oder gleich wie 20 ppm, bezogen auf das Gesamtgewicht des Kohlenwasserstofföls, umfasst; und/oder
- eine Siedetemperatur von 230 bis 340 °C, vorzugsweise 235 bis 330 °C und bevorzugter 240 bis 325 °C gemäß ASTM D86 aufweist; und/oder
- eine biologische Abbaubarkeit nach 28 Tagen von mindestens 70 %, vorzugsweise mindestens 75 % und noch bevorzugter mindestens 80 %, gemessen nach der Norm OECD 306, aufweist; und/oder
- einen Flammpunkt höher als oder gleich wie 110 °C nach der Norm EN ISO 2719 aufweist; und/oder
- durch ein katalytisches Hydrierungsverfahren bei einer Temperatur von 80 bis 180 °C und einem Druck von 50 bis 160 bar aus einem deoxygenierten und/oder isomerisierten Einsatzstoff biologischen Ursprungs erlangt wird.

7. Gelierte Zusammensetzung nach einem der vorherigen Ansprüche, umfassend, bezogen auf das Gesamtgewicht der gelierten Zusammensetzung, 50 bis 99 Gewichts-%, vorzugsweise 60 bis 95 Gewichts-%, bevorzugter 70 bis 90 Gewichts-%, Kohlenwasserstofföl und 1 bis 50 Gewichts-%, vorzugsweise 5 bis 40 Gewichts-%, bevorzugter 10 bis 30 Gewichts-%, des Geliermittels, das vorzugsweise ausgewählt ist aus den gelierenden Polymeren.

8. Kosmetische, dermatologische oder pharmazeutische Zusammensetzung, umfassend mindestens eine gelierte Zusammensetzung nach einem der Ansprüche 1 bis 7, vorzugsweise in einer Menge von 0,5 bis 80 %, bevorzugt von 1 bis 50 % und vorteilhafterweise von 5 bis 30 Gewichts-%, bezogen auf das Gesamtgewicht der Zusammensetzung.

9. Kosmetische, dermatologische oder pharmazeutische Zusammensetzung nach Anspruch 8, umfassend mindestens einen Fettkörper, der ausgewählt ist aus: pflanzlichen Ölen, Kohlenwasserstoffölen, die nicht das Kohlenwasserstofföl der gelierten Zusammensetzung sind, pflanzlichen Buttersorten, Fettethern und -alkoholen, Ölestern, Alkanen und Silikonölen, und/oder mindestens einen Zusatzstoff, der vorzugsweise ausgewählt ist aus den Emulgatoren.

10. Verwendung der gelierten Zusammensetzung nach einem der Ansprüche 1 bis 7 oder der Zusammensetzung nach Anspruch 8 oder 9 zur topischen Anwendung, insbesondere als Haut- oder Haarpflegeprodukt, als Make-up-Produkt, als Haarpflegeprodukt, als Make-up-Entferner, als Lippenpflegeprodukt, wie beispielsweise Lipgloss oder Feuchtigkeitsstifte für die Lippen.

11. Verfahren zur kosmetischen Behandlung der Haut, umfassend mindestens einen Schritt eines Auftragens, vorzugsweise durch Verteilen, der gelierten Zusammensetzung nach einem der Ansprüche 1 bis 7 oder der Zusammensetzung nach Anspruch 8 oder 10.

## Claims

1. Gelled composition comprising:
- at least 50% by weight of at least one hydrocarbon oil that has a content by weight of isoparaffins ranging from 90 to 100%, a content by weight of normal paraffins ranging from 0 to 10% and a content of carbon of biological origin greater than or equal to 90% relative to the total weight of the hydrocarbon oil, said hydrocarbon oil having a biodegradability at 28 days of at least 60%, measured according to the standard OECD 306, and
- at least 0.5% by weight of at least one gelling agent chosen from among:
i. gelling polymers chosen from among homopolymers or copolymers comprising at least one monomer chosen from among isoprene, butadiene, styrene and (meth)acrylates,
ii. non-polymer gelling agents,
relative to the total weight of the gelled composition.

2. Gelled composition according to claim 1, wherein the gelling agent is a gelling polymer chosen from among homopolymers or copolymers comprising at least one monomer chosen from among isoprene, butadiene, styrene and (meth)acrylates, preferably the gelling polymer is chosen from among homopolymers or copolymers comprising at least one monomer chosen from among isoprene, butadiene, styrene, more preferably the polymer is a copolymer comprising at least one styrene monomer and at least one monomer chosen from among ethylene, propylene, butadiene, isoprene.

3. Gelled composition according to claim 2, wherein the polymer is chosen from among star polymers, diblock copolymers, triblock copolymers, multiblock copolymers, comb polymers, radial polymers, and combinations of the latter.

4. Gelled composition according to any of claims 2 to 3, wherein the polymer is chosen from among styrene/butadiene copolymers, styrene/isoprene copolymers, hydrogenated styrene/butadiene copolymers, hydrogenated styrene/isoprene copolymers, hydrogenated polyisoprene crosspolymers, polyisoprene homopolymers, hydrogenated styrene thermoplastic homopolymers, liquid rubbers, and mixtures thereof.

5. Gelled composition according to claim 1, wherein the gelling agent is a non-polymer gelling agent chosen from among:
- mineral fillers chosen preferably from among clays, montmorillonites, silicates, silicas, hectorites,
- waxes chosen preferably from among natural waxes, such as vegetable waxes, animal waxes, ozokerites and ceresins, and
- ammonium salts and metal salts chosen preferably from among zinc stearate.

6. Gelled composition according to any of the preceding claims wherein the hydrocarbon oil:
- is chosen from among non-cyclic isoparaffins comprising from 14 to 18 carbon atoms; and/or
- comprises a content by weight of isoparaffins ranging from 95 to 100% and preferentially from 98% to 100% relative to the total weight of the hydrocarbon oil; and/or
- comprises a content of carbon of biological origin greater than or equal to 95%, preferably greater than or equal to 98% and preferentially of 100%; and/or
- comprises a content by weight of normal paraffins less than or equal to 5% and preferably less than or equal to 2% relative to the total weight of the hydrocarbon oil; and/or
- a content by weight of naphthenic compounds less than or equal to 1%, preferably less than or equal to 0.5% and preferentially less than or equal to 100 ppm relative to the total weight of the hydrocarbon oil; and/or
- a content by weight of aromatic compounds less than or equal to 500 ppm, preferably less than or equal to 300 ppm, preferentially less than or equal to 100 ppm, more preferentially less than or equal to 50 ppm and advantageously less than or equal to 20 ppm, relative to the total weight of the hydrocarbon oil; and/or
- has a boiling temperature ranging from 230 to 340°C, preferably from 235 to 330°C and more preferentially from 240 to 325°C according to the standard ASTM D86; and/or
- has a biodegradability at 28 days of at least 70%, preferably of at least 75% and even more preferably of at least 80% measured according to the standard OECD 306; and/or
- has a flash point greater than or equal to 110°C according to the standard EN ISO 2719; and/or
- is obtained by a method of catalytic hydrogenation at a temperature from 80 to 180°C and at a pressure from 50 to 160 bars of a deoxygenated and/or isomerised feedstock of biological origin.

7. Gelled composition according to any of the preceding claims comprising, relative to the total weight of the gelled composition, from 50 to 99% by weight, preferably from 60 to 95% by weight, more preferably from 70 to 90% by weight, of hydrocarbon oil and from 1 to 50% by weight, preferably from 5 to 40% by weight, more preferably from 10 to 30% by weight, of said gelling agent, preferably chosen from among said gelling polymers.

8. Cosmetic, dermatological or pharmaceutical composition comprising at least one gelled composition according to one of claims 1 to 7, preferably in a quantity ranging from 0.5 to 80%, preferentially from 1 to 50% and advantageously from 5 to 30% by weight relative to the total weight of the composition.

9. Cosmetic, dermatological or pharmaceutical composition according to claim 8, comprising at least one fatty substance chosen from among: vegetable oils, hydrocarbon oils other than the hydrocarbon oil of said gelled composition, vegetable butters, fatty alcohols and ethers, oily esters, alkanes and silicone oils and/or at least one additive preferably chosen from emulsifiers.

10. Use of the gelled composition according to any of claims 1 to 7, or of the composition according to claim 8 or 9 for a topical application, in particular as a care product for the skin or hair, as a make-up product, as a hair care product, as a makeup remover, as a lip care product, such as a gloss or moisturising sticks for lips.

11. Method for the cosmetic treatment of the skin comprising at least one step of applying, preferably by spreading, the gelled composition according to any of claims 1 to 7 or the composition according to claim 8 or 10.
